# EUROPEAN PATENT SPECIFICATION

(11) **EP 2 307 562 B1**
(45) Date of publication and mention of the grant of the patent: **30.10.2013**
(21) Application number: 08874771.2
(22) Date of filing: 17.11.2008
(51) Int. Cl.: C12Q 1/68, C12N 15/79

(54) **EXPRESSION VECTORS BASED ON MODIFIED RIBOSOMAL PROTEIN PROMOTERS AND USES THEREOF IN POST-TRANSCRIPTIONAL ASSESSMENT**
EXPRESSIONSVEKTOREN AUF DER BASIS MODIFIZIERTER RIBOSOMENPROTEIN-PROMOTOREN UND VERWENDUNGEN DAVON BEI DER POSTTRANSKRIPTIONALEN BEURTEILUNG
VECTEURS D EXPRESSION À BASE DE PROMOTEURS DE PROTÉINE RIBOSOMIQUE MODIFIÉS ET UTILISATIONS DE CEUX-CI DANS L ÉVALUATION POST-TRANSCRIPTIONNELLE

(30) Priority: 27.06.2008 WO PCT/EP2008/005278
(43) Date of publication of application: 13.04.2011
(73) Proprietor: King Faisal Specialist Hospital & Research Centre, Riyadh 11211 (SA)
(72) Inventor: KHABAR, Khalid, S., Abu, Riyadh 11211 (SA)
(74) Representative: Engelhard, Markus
(86) International application number: PCT/EP2008/009712
(87) International publication number: WO 2009/155961

(56) References cited:
- WO-A-2005/000888
- WO-A-2006/123097
- US-B1- 6 177 611
- YOSHIHAMA MAKI ET AL: "The human ribosomal protein genes: Sequencing and comparative analysis of 73 genes" GENOME RESEARCH, vol. 12, no. 3, March 2002 (2002-03), pages 379-390, XP002516388 ISSN: 1088-9051
- HAN J ET AL: "ENDOTOXIN-RESPONSIVE SEQUENCES CONTROL CACHECTIN-TUMOR NECROSIS FACTOR BIOSYNTHESIS AT THE TRANSLATIONAL LEVEL" JOURNAL OF EXPERIMENTAL MEDICINE, vol. 171, no. 2, 1990, pages 465-476, XP002516389 ISSN: 0022-1007
- MOOR C H D ET AL: "Mechanisms of translational control by the 3' UTR in development and differentiation" SEMINARS IN CELL AND DEVELOPMENTAL BIOLOGY, ACADEMIC PRESS, GB, vol. 16, no. 1, 1 February 2005 (2005-02-01), pages 49-58, XP004711655 ISSN: 1084-9521
- WILKIE G S ET AL: "Regulation of mRNA translation by 5'- and 3'-UTR-binding factors" TRENDS IN BIOCHEMICAL SCIENCES, ELSEVIER, HAYWARDS, GB, vol. 28, no. 4, 1 April 2003 (2003-04-01), pages 182-188, XP004421226 ISSN: 0968-0004

## Description

The present invention relates to the use of an expression vector comprising (a) a promoter region comprising a non-inducible constitutively active ribosomal protein gene promoter, (b) an operably linked reporter or gene sequence, and (c) a 3' untranslated region (3' UTR), which are suitable means for an selective assessment of post-transcriptional regulation, post-transcriptional control elements and factors as well as for identifying compounds that effect post-transcription. The present invention furthermore relates to the use of arrays, expression vector libraries and cell lines containing the expression vector(s) in a method for identifying compounds that affect post-transcriptional regulation of reporter(s) or gene(s), that utilize the expression vector(s).

### BACKGROUND OF THE INVENTION

Transcriptional regulation occurs as a result of many different signaling pathways that lead to an activation of transcriptional factors that regulate promoter transcriptional activity. Alterations in gene expression leading to transcription and transcriptional regulation can be induced by a wide variety of inducers, stress, insults, environmental changes, and during development and other biological processes such as cellular growth, innate immunity, and metabolism. Reporter gene technology is a widely used and important approach to assess promoter activity or expression and changes in gene expression as a result of transcriptional regulation. The term "reporter" refers to a gene product that can be easily measured when it is fused to transcriptional control elements, such as those in promoters, and that "reports" the effect of a signalling cascade or experimental conditions on gene expression in living cells. The promoter is best known to be located immediately upstream of a transcription start site of a gene and often comprises a core promoter, is generally within 50 bp of the transcription site where the pre-initiation complex forms along with a general transcription machinery which assembles including polymerase II, and an extended promoter that can contain specific regulatory sequences elements (Cooper *et al.,* 2006).

WO2006/123097 discloses vectors comprising a promoter region of a ribosomal protein for the expression of a gene sequence. The assessment of post-transcriptional effects is not disclosed.

WO2005/000888 discloses a vector comprising an RPS21 promoter operably linked to a heterologous nucleic acid sequence and optionally a polyadenelation sequence.

US 6,177,611 discloses the use of a RPS8 promoter for constitutively expressing a heterologues nucleotide sequence in a plant.

Yoshihama Maki et al. (Genome Research, 2002, volume 12, no. 3, pages 379-390) discloses sequencing and comparative analysis of ribosomal protein genes.

Han et al. (Journal of Experimental Medicine, 1990, volume 171 no. 2, pages 465-476) discloses a construct comprising a reporter gene, constitutively transcribed under the influence of a promoter unresponsive to specific factors of interest.

Moor et al. (Seminars in Cell and Developmental Biology 2005, volume 16, no. 1, pages 49-58) discloses mechanisms of translational control by the 3' UTR.

Regulated gene technologies including reporter gene technology is important both in academic basic scientific research and in pharmaceutical industry research. For example, it is used to monitor transcriptional activity of a specific promoter of interest or a cis-acting sequence of interest in health, disease, or therapeutic intervention research setting. The specific promoter of interest can be synthesized *de novo* or purposely altered, for example, by introducing specific mutations. Reporter gene technology is also used to assess the activity of transacting factors on specific promoters or sequences.

Regulated gene technologies including reporter gene technology is widely used in drug industry to discover and assess modulators, e.g., inhibitors, of the activity of specific promoters, specific *cis*-acing sequences, or of *trans-*acting factor activity. A drug discovery assay based on reporter gene technology is based on the measurement of transcription activity of a detectable reporter that is operably linked to promoters or specific *cis*-acting sequence elements that are activated by signaling cascades allowing a screening for compounds that modulate the transcriptional activity. The transcriptional reporter system can be studied for the effect of a new gene product or drug candidate on a particular signal transduction pathway.

Notable examples of cis-acting elements are, but not limited to, NFκB elements, AP-1 elements, IFN-stimulated response elements (ISRE), metal response elements, c-myc response elements, p53 response elements, calcium response elements, and many others. Intracellular receptors such as hormone receptors, e.g. glucocorticoid receptor, estrogen receptor, and androgen receptor, can also mediate transcription and can reach the nucleus either in its native state or modified by a specific signaling event.

Many of the regulatory transcriptional elements are used with reporter assays in a drug discovery program. For the purpose of providing an example, NFκB is one of these important elements in inflammation and cancer. This protein is a member of the rel family of transcription factors that regulate several important physiological processes, including immune responses, inflammation, cell growth, apoptosis, and tumorigenesis. As a result, the NF-κB signaling pathway has been increasingly seen as a promising target for pharmacological intervention, especially in models of inflammation or cancer, where the pathway is often constitutively active (Calzado *et al.,* 2007). Many different stimuli have been identified which activate the NF-κB pathway such as the pro-inflammatory cytokines tumor necrosis factor (TNF-α) and interleukin-1 (IL-1). There are many signaling pathways that regulate transcription that can be targeted by drugs. These signaling events result in modification and activation of transcriptional factors that subsequently act on the transcriptional machinery. Thus, the transcription reporter assays are important in pharmaceutical industry.

However, when post-transcriptional assessment is desired instead of assessing transcriptional effects, different regulated gene technologies including reporter gene technology have to be utilized, namely systems without or with minimal interference of transcriptional effects.

Thus, there is a need in the art for improved means and methods in the field of regulated gene technologies.

Thus, the object of the present invention is to provide means and methods that allow studying (post-)transcriptional regulation, in particular an improved assessment of post-transcriptional effects.

### SUMMARY OF THE INVENTION

The object of the present invention is solved by the subject-matter as defined in the attached claims.

In particular, the object of the present invention is solved by the use of providing an expression vector in accordance with the appended claims, for accessing post-transcriptional effects.

An expression vector according to the present invention comprises
(a) a promoter region comprising a non-inducible constitutively active ribosomal protein gene promoter,
(b) an operably linked reporter or gene sequence, and
(c) a 3' untranslated region (3' UTR).

Also disclosed is the use of the expression vectors according to the present invention for producing an array.

Also disclosed is an array produced according to the present invention that comprises at least an expression reporter or gene construct of the present invention.

Also disclosed is a library of expression vectors comprising at least 2 expression vectors according to the present invention, wherein each expression vector comprises a different post-transcriptional control element.

Also disclosed is a stable cell line that harbors the expression vector according to the present invention, and preferably expresses a reporter or gene protein therefrom.

According to the present invention this object is furthermore solved by a method for identifying compounds that affect post-transcriptional regulation of reporter(s) or gene(s).

A method according to the present invention preferably comprises the following steps:
(1) providing
   at least one expression vector as defined herein,
   at least one linear expression cassette derived from the expression vector as defined herein,
   an array as defined herein,
   a library of expression vectors as defined herein, and/or
   a stable cell line as defined herein,
(2) providing at least a compound to be tested,
(3) determining the effect of the compound on the post-transcriptional regulation by determining the mRNA level and/or the expression level of the reporter or gene.

Also disclosed is a kit for carrying out the method according to the present invention, comprising
(i) at least one expression vector as defined herein,
   and/or
   at least one linear expression cassette derived from the expression vector as defined herein,
(ii) a transfection reagent, and
(iii) an instruction sheet.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

### Expression vectors suitable for assessing post-transcriptional effects

As outlined above, the present invention provides expression vectors comprising
(a) a promoter region comprising a non-inducible constitutively active ribosomal protein gene promoter,
(b) an operably linked reporter or gene sequence, and
(c) a 3' untranslated region (3' UTR).

The expression vector is a nucleic acid construct.

The nucleic acid is preferably DNA, RNA, PNA or comprises modified nucleotides, nucleosides. The expression vector construct can comprise different types of nucleic acids.

### Promoter region (a)

The expression vector according to the present invention comprises a promoter region that comprises a non-inducible constitutively active ribosomal protein gene promoter.

The term "non-inducible constitutively active promoter" refers to a promoter that is constitutively active in a manner that is independent on transcriptional induction.

A non-inducible constitutively active promoter differs from a minimal promoter, because minimal promoters give very weak expression levels and it is difficult to achieve sensitivity and selectivity, in particular with the use of 3'UTR from labile cellular mRNAs, and they are furthermore particularly problematic with the use of drug inhibitors that further attenuate expression levels.

When normally used in regulated gene technology for assessing a transcriptional activation due to specific transacting factors, a promoter linked to specific gene/reporter products is usually minimal to reduce basal background levels and achieve higher transcriptional induction in order to facilitate end measurements. Several minimal promoters are known in the art such as those derived from promoters of the cytomegalovirus (CMV), HSV-1 TK, SV40; and they contain few transcriptional control elements in addition to an RNA polymerase II recognition TATA or TATA-like box. Minimal promoters can also include a CCAAT protein binding site and SP1 site. A minimal promoter includes a TATA box such as TATAAA or its variants. Promoters, either full or partial, can be used to assess transcriptional activation such as in the case of new genes.

However, studies requiring post-transcriptional assessment rely on the use of transcriptional inhibitors that have limitations or minimal promoters that have deficient expression levels.

Now, the inventors developed expression vectors derived from ribosomal protein 23 (RPS23) and ribosomal protein 30 (RPS30) that are transcriptionally non-inducible and constitutively active. Thus, the expression vectors are highly suitable for a number of applications, particularly for selective post-transcriptional assessment. Generally, these ribosomal protein promoters lead to weak expression levels, but, in this patent application, RPS23 and RPS30 were rendered for moderate expression. Unlike CMV and SV40 promoters, the modified RPS30IM system (wherein RPS30IM system refers to RPS30I-M1, RPS30I-M2, RPS30I-M2T, RPS30I-M1TOD as well as RPS30I-M1TOU, see below) was not activated by a number of stimuli and inducers. For example, the RPS30I-M1 system was applied to investigate responses to TNF-α or IL-α in the presence of the phosphatase inhibitor, okadaic acid, known to stabilize AU-rich elements containing-mRNAs and was found to be responsive in a manner that is independent on transcriptional induction. For more details, see herein below, and Figures and Examples.

Preferred ribosomal protein gene promoters of the invention are the promoter of ribosomal protein S23 (RPS23) and ribosomal protein S30 (RPS30), more preferably the human RPS23 promoter or human RPS30 promoter.

The sequences of RPS23 and RPS30 can be found in the RPG ribosomal protein gene database (http://ribosome.miyazaki-med.ac.jp). See also SEQ ID NOs. 1 and 2.
The gene of *Homo sapiens* RPS30 or RPS23, respectively, contain several intron and exon sequence sections as well as a 5' upstream and a 3' downstream region, wherein the promoter region is in the 5' upstream region.

Furthermore preferred are sequences that can be derived from the preferred ribosomal protein gene promoters.

Furthermore, parts or partial sequences of the preferred ribosomal protein gene promoters are also preferred, such as truncated sequences of these promoters, e.g. 5' truncated sequences.

Preferably, truncated sequences that have a length of at least about 100 nucleotides are preferred, such as truncated sequences that have a length of about 100, 150, 200, 250, 300, 350, 400 nucleotides.
In other words, the truncation is of at least 50 nucleotides or about 100 nucleotides, wherein the 5' truncation is preferred. The truncation can also be of 500, 550, 600 or more nucleotides.

Thus, truncations are preferred in a range from about 100 to 1000 nucleotides including all individual integers within that range, wherein the truncation depends on the length of the wildtype or starting sequence. The term "including all individual integers within that range", when used in relation to a range, means, for example, and when e.g. the range is 100 to 500: 100, 101, 102, 103, (...) 496, 497, 498, 500.

For example, RPS30-M1 (SEQ ID NO. 3) is 5'-truncated promoter in which 600 bases were deleted from the 5'end of the wild type promoter sequence (SEQ ID NO. 1) ; RPS30-M2 (SEQ ID NO. 4) is 5'-truncated promoter in which 535 bases were removed from the wild type sequence of SEQ ID NO1.

The inventors have found that these preferred promoters or parts thereof can be modified for higher expression.
The modifications are mutations, deletions, subsitutions of single or several nucleotides, insertion/including of nucleic acid sequences.

The following modifications are preferred:
- modifying the transcriptional initiation sequence,
   preferably by mutating a TATA-like sequence to the TATA signal sequence, more preferably by substituting the TATA-like signal TACAAATA with the TATA signal TATAAATA,
- including at least one sp1 site-containing sequence,
   preferably obtained by truncating the RPS23 promoter or the RPS30 promoter and adding at least one sp1 site-containing sequence.
   In preferred embodiments, two, three, four or more sp1 site-containing sequences are included. Sp1 sites are known in the art. Examples are given herein.

For example,
- RPS30-M1 (SEQ ID NO. 3), which is a 5'-truncated promoter in which 600 bases were deleted from the 5'end of the wild type promoter sequence (SEQ ID NO. 1), has two sp1 sites: TCCCGCCCCGTCCTGCG (position: 230-250 of SEQ ID NO. 3) and GGGGCGGAGC (position: 290-300 of SEQ ID NO. 3).
- RPS30-M2 (SEQ ID NO. 4) is a 5'-truncated promoter in which 535 bases were removed from the wild type sequence of SEQ ID NO1. A 100 bases (position of wild type) that contain additional sp1 site was added: (position of 4-21 of SEQ ID NO. 4 GCCGGGCA TGGTGGCGGG) and (position: 75-87 of SEQ ID NO. 4 GGGAGGC GGAGC). In addition to the following sp1 sites: TCCCGCCCCGTCCTGCG (position: 281-297 of SEQ ID NO. 4) and GGGGCGGAGC (position: 340-49 of SEQ ID NO. 4). Thus, RPS30-M2 contains 4 sp1 sites.
For further details, see below.

The above modifications can also be combined.

The promoter region (a) furthermore preferably comprises one or several of the following
- intron sequence(s) of ribosomal proteins, preferably of RPS30 and/or RPS 23, or parts thereof,
preferably first intron of RPS30 (intron 1 of RPS30) or first intron of RPS23 (intron 1 of RPS23)
The intron sequences act primarily to enhance mRNA accumulation; spliced mRNAs also exhibit higher translational yields than intron-less transcripts.
- exon sequence(s) of ribosomal proteins, preferably of RPS30 and/or RPS 23, or parts thereof,
preferably exon 1 of RPS30 or exon 1 of RPS23
or a part of exon 2 of RPS23 for splicing, such as the first nine nucleotides of exon 2 of RPS23,
- tetracycline operator (tetO) sequences,
The tetracycline operator (tetO; also called tetracycline-responsive element (TRE) or tet-operator) can be located upstream or downstream of the TATA signal or TATA-like signal. The tetO sequences allow for regulation of transcription.
- modified sequences wherein the modification eliminates a restriction site.
such as
a BamH1 site CACTGAG can be eliminated by mutating it into CACCTTGAG,
a ATG can be eliminated by mutating it into CTG.

For preferred combinations of the above described modifications, see below.

The expression vectors according to the present invention preferably comprise a nucleic acid sequence of any of SEQ ID NOs. 3 to 8 or a sequence complementary thereof.
For further details, see below.

### Reporter or gene sequence (b)

The reporter or gene sequence (b) is operably linked to the promoter region (a) as well as the 3'UTR (c), such that, in general, function/activity of the promoter and/or the 3'UTR can be assessed. The transcription, the mRNA level and/or the expression level of the reporter or gene serves as the signal to be measured ("read out").
Herein, the promoter is constitutively active and non-inducible (by transcriptional factors) (with minimal interference from transcriptional effects), thus leading to a transcription and expression of the gene/reporter sequence. Therefore, the expression vectors of the invention allow a selective assessment of post-transcriptional events, such as in response to cellular responses, compounds/potential drug candidates. Also, the 3'UTR and elements comprised therein can be tested with respect to their effect on post-transcription.

Any gene product can be employed as reporter or gene sequence (b) in the expression vectors. Among important gene products are reporters. A reporter that can be employed with this approach is luciferase and β-galactosidase, green and enhanced green fluorescent protein (EGFP), Renilla and firefly luciferases, other luciferases, secreted alkaline phosphatase (SEAP), chloramphenicol acetyltransferase (CAT), secreted hormone, glucose oxidase, secreted cytokine, coral reef fluorescent protein, red and yellow fluorescent proteins, and other fluorescent and bioluminescent proteins. Many companies provide reporter systems including, but not limited to, Promega, Novagen, Clontech, Invivogen, Evrogen, Clontech, Gene Systems, Genelantis, Invitrogen, etc.

In the examples disclosed herein, we used enhanced green fluorescent protein (EGFP) as an example of a reporter. When particularly coupled with advanced imaging processing, it is sensitive and has a large dynamic range. The GFP assay can be performed on living cells allowing repeated monitoring without cell lysis and other manipulations resulting in intra-well variance in fluorescence that is < 6%, which does not warrant intra-well normalization of transfection (Al-Zoghaibi *et al.,* 2007).

Different reporters are known in the art, such as, green fluorescent protein (GFP), luciferase, secreted alkaline phosphatase (SEAP), chloramphenicol acetyltransferase (CAT), secreted hormone, secreted cytokine, β-galactosidase, and other fluorescent and bioluminescent proteins. The choice of a reporter depends on the cell line used (endogenous activity), the nature of the experiment (e.g. dynamics of gene expression and transfection efficiency), and the adaptability of the assay to the chosen detection method (Naylor, 1999). Several modifications of the reporter itself have been sought to improve the reporter performance such as rapid response and magnitude of change, such as the use of destabilization elements (Zhao *et al.,* 1995; Li *et al.,* 1998). Green fluorescent protein (GFP) is increasingly popular because of the possibility of non-invasive monitoring of gene expression in living tissues and cells (Naylor, 1999).

The post-transcriptional activity can also be assayed using the mRNA levels. Real time RT-PCR, Northern blots, RNase protection assays, or any other mRNA or RNA detection and measurement method can be used. Alternatively, protein levels can be assayed when secreted using ELISA or other means as in the case of secreted SEAP and β-galactosidase or by Western blotting as in the case of GFP or other intracellular proteins.
Alternatively, readout and quantitation such us fluorescence and chemoluminescence from reporters, such as GFP or luciferase, respectively, can also be used.

### 3' untranslated region (3' UTR) (c)

The 3' UTR of the expression vector (c) preferably comprises or contains mRNA destabilization or stabilization elements which are derived from a 3' UTR of a cellular mRNA.

Post-transcriptional regulation can be mediated by 3' UTR that harbor mRNA destabilization elements such as AU-rich elements (for a review see Khabar and Young, 2007). Any sequence fragment of 3'UTR can be used which contains sequence elements that negatively or positively affect the post-transcriptional outcome, i.e., at mRNA or protein levels.

mRNA destabilization or stabilization elements are preferably selected from AU-rich elements, GU-rich elements, or U-rich sequences.

The expression vector(s) of the present invention comprise further elements, which are common for expression vectors and are, thus, known to the skilled artisan.

As described above, the present invention provides the use of the expression vector according to the invention for assessing post-transcriptional effects.

The expression vector(s) according to the invention are suitable for assessing post-transcriptional effects with minimal interference from transcriptional effects due to the promoter regions (a) that comprise non-inducible ribosomal protein promoters, in particular the modified ribosomal protein promoters which are modified for higher expression, as described herein.

Thus, the use of the expression vector according to the invention allows the identification of compounds that affect post-transcriptional regulation of genes/reporters.

The expression vectors according to the invention can also be used for producing an array.

### Arrays, libraries and cell lines of the expression vectors

The expression vectors of the invention are versatile means, including, but not limited to
- an array containing functional linear reporter/gene products in which each of the array feature contains a different post-transcriptional factor or regulatory element,
- obtaining a library of expression vectors, wherein each of the expression vectors contains a different post-transcriptional factor or regulatory element,
- obtaining cells that harbour the expression vectors of the invention that can preferably be used for assessing post-transcriptional regulation.

### - Arrays of the expression vector(s)

Also disclosed is an array that can be obtained/produced according to the present invention and that comprises at least an expression reporter or gene construct of the present invention.

An "array" or "microarray" refers to is a multiplex technology used in molecular biology and in medicine. It consists of an arrayed series of several, many or even thousands of microscopic spots of molecules/probes (here: expression reporter or gene constructs), called features. Typically, the molecules/probes are attached to a solid surface by a covalent bond to a chemical matrix. The solid surface can be glass or a silicon chip. Other microarray platforms use microscopic beads, instead of the large solid support. Arrays and microarrays are known in the art.

A preferred array platform/format uses vessels or vessel replicates, such as in microtiter plates.

An array produced according to the present invention comprises at least an expression reporter or gene construct of the present invention, preferably at least 2 expression reporter or gene constructs, in one or more vessels,
wherein each vessel contains an expression reporter or gene construct with a different 3'UTR or post-transcriptional control element(s).

Thus, each expression reporter or gene construct preferably comprises
- a single 3'UTR of cellular mRNA with post-transcriptional element(s),
   or
- a control 3'UTR with introduced post-transcriptional elements, whereas introduced post-transcriptional elements can be inserted by any known method of the art such as by cloning or site-directed mutagenesis.

Wherein the post-transcriptional element(s) are, for example, AU-rich elements, GU-rich elements, or U-rich elements/sequences.

Therefore, preferably each vessel or vessel replicate of the array contains an expression vector with a unique 3'UTR, i.e. the expression vector of each vessel has a different or unique 3'UTR.

A control 3'UTR can be of stable cellular mRNA, such as bovine growth hormone (BGH), rabbit beta-globin, growth hormone 3'UTR.

The arrays of the present invention can comprise further molecules/probes.

### - Libraries of the expression vector(s)

Also disclosed is a library of expression vectors comprising at least two expression vectors according to the present invention, wherein each expression vector comprises a different post-transcriptional control element.
Thus, each member (=expression vector) of the library differs from the other members (=expression vectors) at least by its post-transcriptional control element(s).

Thus, each expression vector of the library preferably comprises
- a single 3'UTR of cellular mRNA with post-transcriptional element(s),
   or
- a control 3'UTR with introduced post-transcriptional elements, whereas introduced post-transcriptional elements can be inserted by any known method of the art such as by cloning or site-directed mutagenesis.

Wherein the post-transcriptional element(s) are, for example, AU-rich elements, GU-rich elements, or U-rich elements/sequences.
A control 3'UTR can be of stable cellular mRNA, such as bovine growth hormone (BGH), rabbit beta-globin, growth hormone 3'UTR.

### - Cell lines of the expression vector(s)

Also disclosed is a cell line that harbours the expression vector according to the present invention, and preferably expresses a reporter or gene protein therefrom.

The cell line is preferably a stable cell line.

Preferably, the expression vector comprises a single 3'UTR of cellular mRNA or a control 3'UTR with post-transcriptional elements, such as AU-rich elements, GU-rich elements, or U-rich elements. A control 3'UTR can be of stable cellular mRNA such as BGH, rabbit beta globin, growth hormone 3'UTR.

In a preferred embodiment, the single cells of the cell line differ from each other in that they harbour expression vectors that differ in their post-transcriptional control element(s).

In a preferred embodiment of the present invention, each cell line harbours an expression vector that comprises a different 3'UTR or post-transcriptional control element(s) from the expression vectors of other cell lines.

Versatility of reporter systems allows use in many applications, for example, but not limited to, drug discovery, drug target discovery, bioassay development, bioassays, cytokine bioassays, interferon response bioassays, virus response bioassays, metal response bioassays, stress response bioassay, inflammatory response bioassays, cell growth assay, cellular behavior indicator assays, angiogenesis bioassay, chemotaxis and metastasis assays, hypoxia assays, environmental changes bioassays using parameters, such as heat, nutrient, radiation, oxygen, pH, salts, toxins. Additionally, any bioassay for inhibition of above responses is also a potential application.

### Method and kit for compound identification

As disclosed above, the present invention furthermore provides a method for identifying compounds that affect post-transcriptional regulation, such as of reporter(s) or gene(s).

Herein, the expression vector(s), arrays, libraries of expression vectors and stable cell lines are valuable tools.

In an embodiment, linear expression cassette(s) derived from the expression vector(s) of the present invention are used. For a method of obtaining such a linear expression cassette, please see below and International patent application PCT/EP2008/005278 of June 27, 2008, which is disclosed herein in its entirety by reference.

Thus, in a first step of the method,
- at least one expression vector as defined herein,
- at least one linear expression cassette derived from the expression vector as defined herein,
- an array as defined herein,
- a library of expression vectors as defined herein, and/or
- a stable cell line as defined herein,
is provided.

In another step, the compound(s) to be tested are provided.

In a further step, the effect of the compound on the post-transcriptional regulation is determined.

This is achieved by detecting and determining and (optionally) quantifying the mRNA level and/or the expression level of the reporter or gene.

Thus, the method according to the present invention preferably comprises the following steps:
(1) providing
   at least one expression vector as defined herein,
   at least one linear expression cassette derived from the expression vector as defined herein,
   an array as defined herein,
   a library of expression vectors as defined herein, and/or
   a stable cell line as defined herein,
(2) providing at least a compound to be tested,
(3) determining the effect of the compound on the post-transcriptional regulation by determining the mRNA level and/or the expression level of the reporter or gene.

Also disclosed is a kit for carrying out the method according to the present invention.

Such a kit comprises preferably
(i) at least one expression vector as defined herein,
   and/or
   at least one linear expression cassette derived from the expression vector as defined herein,
(ii) a transfection reagent, and
(iii) an instruction sheet.

Studies requiring post-transcriptional assessment rely on the use of transcriptional inhibitors that have limitations or minimal promoters that have deficient expression levels. Here, we develop an expression vector derived from ribosomal protein 23 (RPS23) and RPS30 that are transcriptionally non-inducible and constitutively active. Thus, it is suitable for a number of application, particularly for selective post-transcriptional assessment. Generally, these ribosomal protein promoters lead to weak expression levels, but, in this patent application, RPS23 and RPS30 were rendered for moderate expression. Unlike CMV and SV40 promoters, the modified RPS30IM was not activated by a number of stimuli and inducers.

For example, the RPSI30M system was applied to investigate responses to TNF-α or IL-α in the presence of the phosphatase inhibitor, okadaic acid, known to stabilize AU-rich elements containing-mRNAs and found responsive in a manner that is independent on transcriptional induction.

### Production of the expression vectors of the invention

The promoters with their introns are preferably amplified from genomic DNA using PCR with primers specific to the flanking region of each promoter/intron sequence. The primers used include the restriction sites, EcoRV and SalI sites and the resultant PCR products are cloned into an existing reporter expression vector, such as an EGFP expression vector, that was previously cut by EcoRV and SalI. Ribosomal proteins promoters can be amplified from genomic DNA of THP-1 cell line.

Other preferred methods for the production of the expression vectors of the invention is disclosed in the inventors International patent application PCT/EP2008/005278 (corresponds to WO 2009 155950) of June 27, 2008.

These methods are also suitable for obtaining linear expression cassettes derived from the expression vectors of the present invention, such as used in the method and kit for identifying compounds that affect post-transcriptional regulation

In particular, International patent application PCT/EP2008/005278 (corresponds to WO 2009 155950) discloses a method comprising the generation of an expression active linear reporter construct which is controllable or, in other words, regulatable or tunable. This is performed by manipulating the sequence information in the forward primers which contain two regions, a 3' end region that is directed to the vector and can be "vector position-flexible", and a 5' end region that contains *cis*-acting inducible or repressible elements.

International patent application PCT/EP2008/005278 provides a simpler cloning-free method that utilizes PCR with a specific sequence design of the forward primer along with a universal reverse primer, which will be described in details.

Described therein is a simple cloning-free PCR-based procedure to generate gene and reporter expression cassettes that can be used in many applications in the field of life sciences. This cloning-free approach allows promoter activity assessment or transcriptional manipulations including the use of *cis*-acting sequences, that otherwise require cloning and time demanding manipulations, particularly in the case of introduced mutations. In particular, a cloning-free approach to generate transcriptionally controllable linear expression active DNA is described. The expression active linear DNA produced by PCR harbors a part of a promoter, a non-inducible or minimal promoter and contains full functional gene or reporter expression cassettes that include the gene or reporter cDNA and 3'UTR. Transcriptional control elements, such as cis-acting elements, or their mutant forms from several bases to 140 or 200 bases are simply appended to a common DNA sequence in a forward PCR primer that targets the upstream region of the gene or reporter gene of choice, such as reporters, by a single PCR or more, if required. With two- and three-step PCRs, one can generate a 400 bases promoter or transcriptional regulatory elements, if required. The forward 5' upstream primer is versatile in its nucleotide composition, any transcriptional element or regulatory element can be added including mutations and polymorphisms. The described regulated linear reporter gene approach, i.e. introducing a transcriptional control in the linear gene PCR product is simple, versatile and adaptable to high throughput studies that are important in both academic and pharmaceutical research and development activities including drug discovery processes. The invention can be used with any different applications in the field of life sciences including, but not limited to, drug screening, drug target screening, research tool in molecular and cell biology, personalized medicine, pharmacogenomics, and correlation of genetic variations and polymorphisms with phenotypic outcomes.

The principal advantage of the invention disclosed in International patent application PCT/EP2008/005278 is the ease of introducing a transcriptional control of gene or reporter construction, ease of sequence manipulations such as mutations and deletions, reliability, and adaptability to large scale experiments and high throughput drug screening. The assay is dependent on amplification of a functional reporter expression cassette from an optimized mammalian expression vector that is efficient when transfected as PCR product to express the protein of interest. The desired promoter sequences such as a minimal promoter with or without wild type and mutant cis-acting sequences are included in the forward primer that contains at the 5' end several nucleotides targeting a common region upstream of the gene or reporter cDNA. The sequences in the forward primer binding to a common region in the vector or source DNA can be as little as 6 nucleotides. The preferred length is more than 10 bases, particularly preferred is more than 12 bases. The source DNA which can be used for amplification can be the vector itself, a linearized vector that contains the whole functional gene product or reporter cassette, or a linear DNA generated by PCR that contains the whole functional gene product or reporter.

The inventor has used that invention with several different strategies. All of the assays employ a universal reverse primer that target the vector DNA downstream of 3' UTR and a forward primer that targets a region upstream of the reporter construct, depending on the application. In all of the strategies, each PCR is used with one forward and one universal reverse primer. The reverse primer is a universal primer that targets a region downstream of a polyA signal that is sufficient for optimal expression. The preferred distance from the polyA signal is at least 5 nucleotides, particularly preferred is more than 13 bases, and more preferred is more than 20 bases. The specific sequences in the universal primer binding to a vector or source DNA can be as little as 6 nucleotides. The preferred length is more than 10 bases, particularly preferred is more than 14 bases.

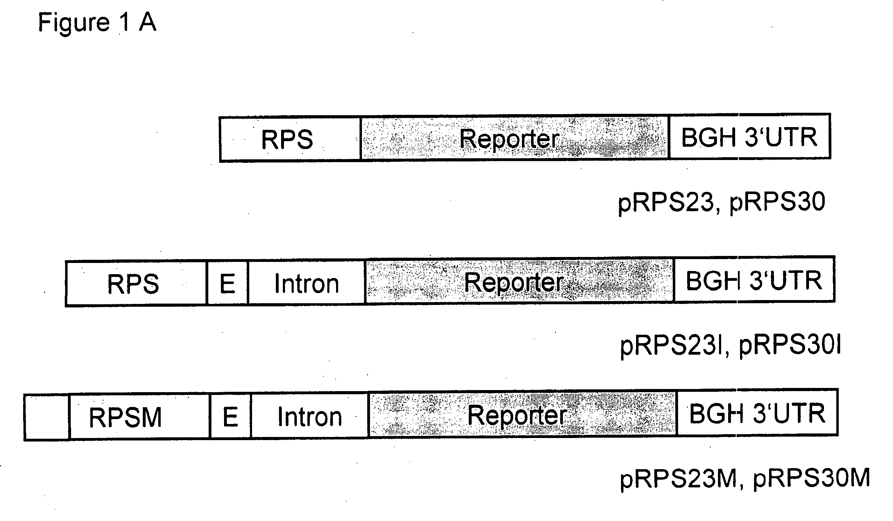

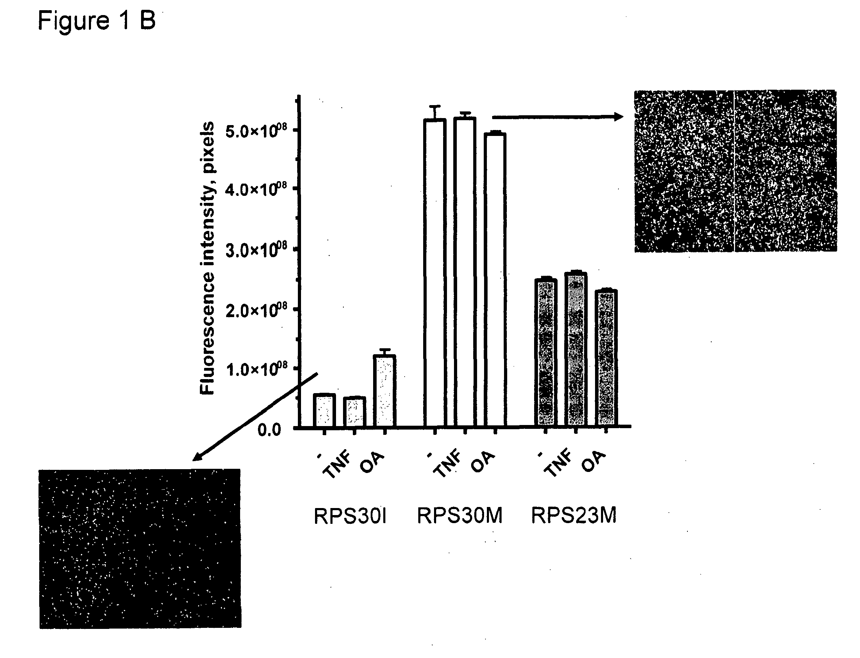

The assay is dependent on amplification of a functional gene product including a reporter expression cassette from an expression vector or DNA source that, when transfected as a linear product, is efficient to express the reporter (Fig. 1). The DNA source can be an expression vector or a fragment of the expression vector. The fragment of the expression vector should harbor the expression cassette composed of a promoter, a cDNA of the gene of interest, and a polyA signal. The vector or plasmid can be produced in abundant amounts using bacterial cultures. The fragment can be linearized by restriction fragments flanking this expression cassette. Alternatively, the expression cassette can be produced by PCR with primers flanking the expression cassette.

An optimized protocol for generating PCR products (100 µl volume) of the various embodiments is as follow: template (100-200 ng), 1X PCR buffer (1.5 mM MgCl₂), 200 µM dNTPs, Hot Start Taq, 2.5 U per reaction, pfx polymerase, 0.20 U per reaction, 0.5 µM final primer concentrations, and the following cycling conditions: 95°C for 12 min and 32 cycles of: 94°C for 30 sec, 53°C for 30 sec, and 72°C for 3.5 min, and final extension 72°C for 7 min. The PCR products should be purified using, for example, Qiagen PCR purification columns.

Any gene product can be employed with the embodiments described therein. Among important gene products are reporters. A reporter that can be employed with this approach is luciferase and β-galactosidase, green and enhanced green fluorescent protein (EGFP), Renilla and firefly luciferases, other luciferases, secreted alkaline phosphatase (SEAP), chloramphenicol acetyltransferase (CAT), secreted hormone, glucose oxidase, secreted cytokine, coral reef fluorescent protein, red and yellow fluorescent proteins, and other fluorescent and bioluminescent proteins. Many companies provide reporter systems including, but not limited to, Promega, Novagen, Clontech, Invivogen, Evrogen, Clontech, Gene Systems, Genelantis, Invitrogen, etc.

In the examples disclosed therein, the inventor used enhanced green fluorescent protein (EGFP) as an example of a reporter. When particularly coupled with advanced imaging processing, it is sensitive and has a large dynamic range. The GFP assay can be performed on living cells allowing repeated monitoring without cell lysis and other manipulations resulting in intra-well variance in fluorescence that is < 6%, which does not warrant intra-well normalization of transfection (Al-Zoghaibi *et al.,* 2007). Also in some of the experiments, the inventor used the dual luciferase system using pGL plasmids that code for firefly and Renilla luciferase reporters from Promega, Inc.

The (post-)transcriptional activity due to the reporter or the gene of interest can also be assayed using the mRNA levels. Real time RT-PCR, Northern, RNase protection assay, or any other mRNA or RNA detection and measurement method can be used. Alternatively, protein levels can be assayed when secreted using ELISA or other means as in the case of secreted SEAP and β-galactosidase or by Western blotting as in the case of GFP or other intracellular proteins.

The desired promoter sequences are added to a forward primer that contains several nucleotides targeting a common region upstream of the reporter or gene cDNA and flexible 5' sequences representing the desired transcriptional factor site, its variants, or any other transcriptional control elements. Transcriptional control elements can be constitutive, inducible or repressible. A universal reverse primer that is specific to the vector DNA downstream of a stable 3' UTR is used in conjunction with the forward primer in the PCR. We have used this approach with several different strategies; each is described herein with a demonstration of its utility with several applications.

The invention disclosed in International patent application PCT/EP2008/005278 is versatile in that it can be used to generate any minimal promoter, a portion of a promoter, an enhancer, positive or negative cis-acting sequences, inducible or repressible control elements, and 5' UTR sequences that are upstream of the gene, or a reporter. An example of a minimal promoter is the CMV minimal promoter which contains an SP1 site (reversed), CAAT (reversed), GC box, and TATA signal. Another example is the HSV-1 TK minimal promoter which contains CCAAT (inverted), SP1, GC-box, and TATA signal. The SV40 minimal promoter is another example. Moloney murine leukemia virus promoter (LASN) is another example. Strong promoters can be derived from housekeeping genes that are abundant, for example, but not limited to, eukaryotic elongation factor alpha (EEF1A1), actin gamma, actin beta, GAPDH, ribosomal proteins, etc. A list of housekeeping genes with their mRNA levels can be found in Eisenberg and Levanon (2003). Any minimal promoter can be derived from any strong promoter. For example, the following sequence tatataat may constitute a minimal promoter.

There are several embodiments that describe various variations of the PCR-based cloning-free generation of transcriptionally-controlled linear reporter constructs.

The forward primer may target a region that contains the minimal promoter. This region should be at least several nucleotides and leads to amplification of a minimal promoter. Thus, the amplification product would include the entire desired minimal promoter which itself may be as minimum as the TATA signal itself, TATAAA. Preferably, the minimal region is longer than 10 bases, more preferable is more than 15 bases, and more preferably is more than 20 bases. The position of the complementary sequences of the forward primers can be flexible allowing the researcher to choose any part of the promoter desired to be part of the resultant amplified product. This shows the tremendous flexibility and universality of the method.

Introduced sequences in the forward primer can be anywhere from several nucleotides to 140 or 150 bases since the longest possible chemically synthesized oligonucleotide that is economically attractive is 150 bases. Many companies provide oligonucleotides having a length up to 100 bases such as Sigma, Operon, and Invitrogen, and some provide up to 140 bases such as Thermo Scientific, Inc. With two- and three-step PCRs, one can generate a 400 bases promoter or transcriptional regulatory elements, if required.

The introduced sequences in the forward primer can be from a group of transcriptional factor or regulatory element sites. These sites can have a wide range of length with a minimum length of few nucleotides. Through this process they control and regulate gene expression. A notable example of cis-acting elements are, but not limited to, NF-κB elements, AP-1 elements, IFN-stimulated response elements (ISRE), metal response elements, c-myc response elements, p53 response elements, hypoxia induced factor response elements, retinoic acid response elements, glucose response elements, calcium response elements, and many others. Intracellular receptors such as hormone receptors, e.g. glucocorticoid receptor, estrogen receptor, and androgen receptor, can also mediate transcription and can reach the nucleus and bind to specific sites in the promoter, either in its native state or modified by a specific signaling event. These sequences bind a transcriptional factor and modulate gene expression. There are both positive regulatory elements such as NF-κB but also negative regulatory elements such as IFN-regulatory factor (IRF2). Examples of the forward primer sequences and examples of their utility in the PCR are disclosed in International patent application PCT/EP2008/005278.

The introduced sequences can be mutated either by a single base mutation mimicking a single nucleotide polymorphism in promoters and regulatory elements. Genetic manipulations such as deletions, insertions, and bases changes can also be part of the introduced sequences. Mutations with one or more bases can provide a control sequence for the transcriptional factor being studied. Examples are "G"→"C" substitution in the NFκB/Rel DNA binding region. The IFN-stimulated response element (ISRE), can be mutated. The introduced sequences can have progressively deleted lengths in order to map a specific regulatory element associated with a specific experimental condition. Examples are given in International patent application PCT/EP2008/005278

Multiple copies of the *cis*-acting enhancer element can be inserted into each construct upstream of a minimal promoter using the simple cloning-free PCR method. Despite the fact that in the examples disclosed in International patent application PCT/EP2008/005278, only two copies of NF-κB were used and one copy of ISRE was used, the response kinetics of the reporter assay can be improved through the use of more copies (Lai *et al.,* 2006) and the use of de-stabilized forms of reporters (Li *et al.,* 1998; Voon *et al.,* 2005). Since the assays were performed with only one or two copies of ISRE or NF-κB, it is expected that increasing copies of these sites may result in stronger induction. In this assay, specific response elements are inserted upstream of a reporter gene. The reporter gene construct is then introduced into cells, either by transfection or electroporation. Extracellular ligands stimulate the activation of specific transcriptional factors, which will then bind to their response elements in the construct and initiate the transcription of the reporter gene or any other gene of interest. By measuring the expression of the gene including reporter genes, the activity of a specific signaling pathway under the influence of a specific drug can be monitored and quantified.

There are several embodiments disclosed in International patent application PCT/EP2008/005278 that reflect variations and the versatility of that invention in modifying the transcriptional activity in promoter studies. In one embodiment, a whole minimal promoter to be studied can be inserted using PCR in which the second part (5' end) segment of a forward primer contains introduced sequences of question to test its ability to evoke the reporter transcription and subsequently the reporter activity. In this way, any small region from the human genome or transcriptome can be used. Examples of the minimal promoters that were used as examples are disclosed in International patent application PCT/EP2008/005278. These minimal promoters can be constitutive such as, but not limited to, ribosomal protein promoters, constitutively active cellular promoters such as elongation factor promoter, actin promoters, and many others. Examples of ribosomal protein promoters are, but not limited to, RPS2 and RPL39. Examples of cellular promoters are, but not limited to, elongation factor and actin gamma promoters. Minimal promoters can also be inducible such as, but not limited to, IFNB minimal promoter which contains virus induced elements and metal response elements (Fig. 7). Examples of forward primes containing minimal promoters of constitutive and inducible elements are disclosed in International patent application PCT/EP2008/005278.

In another embodiment, negative regulatory elements such as those that bind to a transcriptional repressor that affects transcription of the reporter can be included in the forward 5' end region. A forward primer contains 3' end sequences that target a fixed region in the vector template that is upstream of a desirable promoter or promoter fragment and 5' end sequences that are flexible to accommodate desired *cis*-acting negative regulatory sites. The reverse universal primer targets a region downstream of a polyA site. Purified PCR products are transfected into mammalian cells to express the reporter and to assay for its expression or activity under the desired experimental conditions. In this embodiment, it is desirable to have a strong promoter in order to achieve an acceptable sensitivity when transcription is reduced by negative regulatory elements. Examples of strong promoters are CMV promoter, SV40 promoter, β-actin promoter, elongation factor promoter, and many others (Yew *et al.,* 1997; Xu *et al.,* 2001). The construct used herein has an intron. Enhancer and introns are known to enhance expression of transgenes but this may be dependent on the promoter, intron of a specific gene, and the cell line. Examples of introns are CMV intron A and rabbit β-globin introns. Expression can also be enhanced by using strong polyadenylation signals such as those derived from bovine growth hormone (BGH), rabbit β-globin gene, and SV40 polyadenylation signals.

Within the use of this embodiment, there are many negative response elements. For example, it has been shown that a negative regulatory control element is found upstream of NF-κB and can cause repression of TNF induction (Fong *et al.,* 1994). Thus, the 5' end region in a forward primer can include a negative response element such as the TNF promoter. Other examples of negative response elements known in the field are: negative cyclic AMP (cAMP) response elements, retinoic acid response elements, negative interferon regulatory elements, negative hormone response element, NF-κB negative response elements, and many others. Some of the transcription sites that are positive regulatory transcriptional factors can act as negative response elements for other factors, such as the repressor interferon-response factor (IRF-2) overlaps with IRF-1 sites that are positive regulatory sites for interferon response (Paun and Pitha, 2007).

In another embodiment, it may be desirable to use a longer promoter sequence that is non-inducible or constitutively active and then to append the promoter in the PCR to the forward primer that contains any desired sequence or variant. Fig. 11 of International patent application PCT/EP2008/005278 shows examples of the cellular promoter and ribosomal promoters that are not activated with TNF-α, a pro-inflammatory inducer of NF-κB transcriptional factor, unlike CMV and SV40 promoters that can be activated by pro-inflammatory cytokines. In certain occasions, it may be desirable to append specific sequences to a promoter structure of more than a minimal promoter. As an example, we have constructed several vector constructs that harbor several housekeeping gene promoters, primarily ribosomal protein promoters, of which gene products are known for their increased abundance and lack of common inducible transcriptional sites such as NF-κB. We have used these to pinpoint the optimal promoter in the PCR product that gives the most sensitive detection of the EGFP reporter. The RPS23 gave the best promoter with the linear DNA product obtained by PCR using a forward primer in which its 3' end part is specific for a common vector sequence and the same universal reverse primer. As an example, the 5' end of the forward primer contains NF-κB or NF-κB non-responsive. Any regulatory transcriptional control elements can be appended in the forward 5' end part. Examples of these transcriptional control elements are given therein.

Any combination of promoter and cDNA of interest can be used with the invention disclosed in International patent application PCT/EP2008/005278. For example, SV40 minimal promoter and the luciferase reporter are used to generate a transcriptionally-controlled expression active PCR product. The forward primer can incorporate transcriptional control elements such as NF-κB and a mutant control sequence.

In another embodiment disclosed in International patent application PCT/EP2008/005278, a non-inducible promoter such as a minimal promoter or a larger non-inducible promoter can be used to construct a linear cassette with a controlled inducible reporter or any other gene product. There are many other examples of inducible elements that can be used in this strategy, i.e., controlled expression of the reporter or the gene activity. These are, but not limited to, metal response elements, heat shock response, isopropyl beta-D-thiogalactoside (IPTG), and hormone response elements such as ponasterone A induction of the EcP system and dexamethasone-MMLV promoter system (Meyer-Ficca *et al.,* 2004). An example of hormone receptor technology is the one available from New England Biolabs by induction by a synthetic inducer, RheoSwitch Ligand RSL1, and a chimeric bipartite nuclear receptor. Other examples are the Q-mate expression system which is available from Q-biogene, in which repression of gene expression is mediated by the cumate repressor protein CymR bound to operator sites in the absence of the inducer molecule cumate. A common technology relates to is the tetracycline-responsive elements which are regulated by the repressor rtTA or the activator protein tTA, the Tet-Off and Tet-On systems, respectively (Gossen and Bujard, 2002). Doxcycline or tetracycline to turn on or off genes of interest including the reporter can be used. For example, the tetracycline-responsive positive element can be included in the 5' end of the forward primer. The resultant PCR product harboring the reporter or the gene of interest will be active or inactive (depending on whether the response element is on or off). Then, these products are transfected into cells that express either an activator or repressor, and the gene expression or activity is monitored. Another example of inducible systems is given; the disclosed approach was easily applied to generate in a cloning-free manner a PCR product expressing a gene under the control of metal responsive transcription. The metal response elements of metallothionein, MT1G and MT1G, were included in the forward primer. The PCR product was able to express the gene product EGFP reporter, when the heavy metal cadmium was used. Other metals known in the art such as zinc and copper can also be used as inducers.

The disclosed example of converting the EGFP reporter is an important embodiment of the described method of covering any expression construct harboring a gene, cDNA, peptide sequence, small inhibitory RNA sequence, or any other desired sequence into a inducible/repressible expression system. Inducible/repressible systems are important research tools particularly in the case of toxic polypeptides.

In one embodiment, the invention of International patent application PCT/EP2008/005278 can also be applied to study enhancers and 5' UTRs regulatory sequences that exist upstream of the gene. Tissue specific sequences can also be added to any of the embodiments disclosed herein. Tissue specific sequences allow the expression only in a specific tissue or cell type. For example, but not limited to, a liver specific response element can be appended to the 5' end of the forward primer to generate an expression active PCR product that is active in liver cells or hepatoma cell lines. An example of a forward primer that harbors a liver specific transcriptional site for a liver specific factor is disclosed in International patent application PCT/EP2008/005278. There are many examples of transcriptional sequence elements that are tissue-specific including, but not limited to, breast tissues, cardiac tissues, nerve tissues, gland specific tissues such as thyroid and prostate tissues, and many others.

In all above embodiments, any genetic variation can be easily swapped in the forward primer including, but not limited to, single nucleotide polymorphism, mutations of more than one nucleotide, a deletion or insertion in a specific transcriptional region, tandem arrangements of the *cis*-acting elements and position variations.

Stable reporter cell lines can be generated by using a vector or linear DNA cassette that contains a selectable marker. Selectable markers are, but not limited to, neomycin, blasticidin, puromycin, zeocin, hygromycin, and dihydrofolate reducatase (dhfr). Because of the ease of making the reporter linear construct with the desired introduced sequences or variations, it is also expected that the generation of a stable reporter cell line is also simple. In this case, co-transfection with a plasmid or PCR product coding for the selection marker that contains the expression cassette of a selection marker is necessary.

The primary goal of the invention of International patent application PCT/EP2008/005278 was to provide a simple method for producing transcriptional control elements and transcriptional manipulations of a reporter gene as linear DNA constructs for *"in vivo"* applications, i.e., using assays based on living cells. Specifically, the present invention's aim is to provide a simple method for manipulating promoter and transcriptional control and regulatory elements including introduced mutations and genetic variations, without the need for the time-demanding cloning steps. The method is also useful for identifying and analyzing new *cis-* and *trans*-acting regulatory sequences/factors as well as is particularly useful for drug screening and drug discovery.

The assessment and measurement of the reporter activity can be approaches, not only of the activity of the reporter proteins, but also of the levels of the reporter proteins. Reporter levels, whether intracellular or secreted, can be measured by any detection method including Western blotting, colorimetric method, fluorescence, luminescence, biosensors, and many others. Also, mRNA levels of the reporter can be used to monitor the transcription of the promoter. The mRNA levels can be assessed and quantified by a variety of techniques including, but not limited to, semi-quantitative PCR, real-time PCR, Northern blotting, RNase protection assay, beads-dependent mRNA quantification, *in situ* hybridization, and others. Examples of fluorescence, luminescence, and mRNA levels are disclosed in International patent application PCT/EP2008/005278.

In general, reporters that produce a detectable signal either directly such as a fluorescent signal, a change in absorbance, and a phosphorescent signal, or indirectly by labeling with a conjugate such as a chemical substrate, antibody, or ligand can be used. A signal produced by the reporter can be detected by many methods including, but not limited to, spectroscopic, spectrophotometric, biochemical, immunochemical, electrical, photochemical, optical, thermal, pH or chemical means, visual inspection, or structural and biophysical characteristics.

Because of the ease of producing the linear reporter and introducing desired variations leading to the transcriptional control, one may expect to produce a high throughput array composed of these linear reporters harboring the different transcriptional control elements and their variations such as mutations. Nowadays, there are many high-throughput automation systems that facilitate the process including cell dispensing, transfection, and detection systems. Detection systems such as imaging of fluorescent reporters are those such as the automated imagers available from BD imaging systems (BD Dickinson, Inc.), Genetix, and Cellomics. Microplate readers and array scanners can also be applied in high throughput applications.

Thus, the method allows a versatile number of applications including, but not limited to, making a library of transcriptionally regulated functional linear reporter or gene PCR products, an array containing functional linear reporter PCR products in which each of the array feature contains a transcriptional factor or regulatory element, and a kit that contains the necessary reagents to construct the linear reporter PCR product.

Versatility of reporter systems allows use in many applications, for example, but not limited to, drug discovery, drug target discovery, bioassay development, bioassays, cytokine bioassays, interferon response bioassays, virus response bioassays, metal response bioassays, stress response bioassay, inflammatory response bioassays, cell growth assay, cellular behavior indicator assays, angiogenesis bioassay, chemotaxis and metastasis assays, hypoxia assays, environmental changes bioassays using parameters, such as heat, nutrient, radiation, oxygen, pH, salts, toxins. Additionally, any bioassay for inhibition of above responses is also a potential application.

In another embodiment disclosed in International patent application PCT/EP2008/005278, the use of non-inducible or regulatable, such as with tetracyclines, minimal promoters in post-transcriptional assessment without or with minimal interference of transcriptional assessment is disclosed therein. Specifically, the solution is given to a problem where post-transcriptional assessment is desired instead of transcriptional effects. Post-transcriptional regulation can be mediated by 3' UTR that harbor mRNA destabilization elements such as AU-rich elements (for a review see Khabar and Young, 2007). Any sequence fragment of 3'UTR can be used which contains sequence elements that negatively or positively affect the post-transcriptional outcome, i.e., at mRNA or protein levels. In the case of ribosomal protein promoters, the preferred embodiment for post-transcriptional regulation is the vectors themselves rather than the PCR product.

The various embodiments of the described technology in the entire International patent application PCT/EP2008/005278 can be applied in animals studies, such as transgenic mice or small inhibitory RNA transgenic mice.

### Ribosomal protein promoter sequences and preferred modifications

### 1. RPS30

The starting sequence that the inventors used for their promoter modifications can be found on the RPG ribosomal protein gene database
(http://ribosome.miyazaki-med.ac.jp/rpg.cgi?mode=strc&id=HUM10033).
See also SEQ ID NO. 1.
The gene of *Homo sapiens* RPS30 contains several intron and exon sequence sections as well as a 5' upstream and a 3' downstream region. The putative transcriptional initiation box is tacaaata (underlined).

### 5' Upstream

### Exon 1

CTCTTTCTCGACTCCATCTTCGCGGTAGCTGGGACCGCCGTTCAG

### Intron 1

### Exon 2

### Intron 2

### Exon 3

### Intron 3

### Exon 4

GTAAAGTCCATGGTTCCCTGGCCCGTGCTGGAAAAGTGAGAGGTCAGACTCCTAAG

### Intron 4

### Exon 5

### 3' Downstream

### 2. RPS23

The starting sequence that the inventors used for their promoter modifications can be found on the RPG ribosomal protein gene database
(http://ribosome.miyazaki-med.ac.jp/rpg.cgi?mode=strc&id=HUM10025).
See also SEQ ID NO. 2.
The gene of *Homo sapiens* RPS23 contains several intron and exon sequence sections as well as a 5' upstream and a 3' downstream region.

### 5' Upstream

### Exon 1

CTCTTTCGCTCAGGCCCGTGGCGCCGACAGGATGG

### Intron 1

### Exon 2

### Intron 2

### Exon 3

### Intron 3

### Exon 4

### 3' Downstream

### 3. RPS30I-M1

### SEQ ID NO. 3

### Modifications

1. 5' truncated RPS30 promoter (+from transcriptional start site),
2. 60 bases were added that include sp1 site,
3. RPS30 exon 1,
4. first intron of RPS30 , and
5. nine bases of exon 2 RPS23 for splicing.
6. The putative transcriptional initiation box was improved from tacaaata to tataaata.
7. BamH1 site CACTGAG was eliminated by mutating to CACCTTGAG.

RPS30-M1 (SEQ ID NO. 3) is a 5'-truncated promoter in which 600 bases were deleted from the 5'end of the wild type promoter sequence (SEQ ID NO. 1). RPS30-M1 has two sp1 sites: TCCCGCCCCGTCCTGCG (position 230-250) and GGGGCGGAGC (position 290-300).

### 4. RPS30I-M2

### SEQ ID NO. 4

### Modifications

1. 5' truncated RPS30 promoter (+from transcriptional start site),
2. 100 bases were added that include sp1 site,
3. RPS30 exon 1,
4. first intron of RPS30 , and
5. nine bases of exon 2 RPS23 for splicing.
6. /
7. BamH 1 site CACTGAG was eliminated by mutating to CACCTTGAG

RPS30-M2 (SEQ ID NO. 4) is a 5-truncated promoter in which 535 bases were removed from the wild type sequence of SEQ ID NO1. A 100 bases (position of wild type) that contain additional sp1 site was added: (position of 4-21 GCCGGGCA TGGTGGCGGG) and (position 75-87 GGGAGGC GGAGC). In addition to the following sp1 sites: TCCCGCCCCGTCCTGCG (position: 281-297) and GGGGCGGAGC (position 340-49). Thus, RPS30-M2 contains 4 sp1 sites.

### 5. RPS30I-M2T

### SEQ ID NO. 5

### Modifications

1. 5' truncated RPS30 promoter (+from transcriptional start site),
2. 100 bases were added that include sp1 site,
3. RPS30 exon 1,
4. first intron of RPS30 , and
5. nine bases of exon 2 RPS23 for splicing.
6. The putative transcriptional initiation box was improved from tacaaata to tataaata.
7. BamH1 site CACTGAG was eliminated by mutating to CACCTTGAG.

A 100 bases (position of wild type) that contain additional sp 1 site was added: GCCGGGCA TGGTGGCGGG and GGGAGGC GGAGC. In addition to the following sp1 sites: TCCCGCCCCGTCCTGCG and GGGGCGGAGC. Thus, RPS30-M2T contains 4 sp1 sites.

### 6. RPS23I-M

### SEQ ID NO. 6

### Modifications

1. 5' truncated promoter (+392 from transcriptional start site),
2. addition of 68 bases of Sp1 site-containing sequences of RPS23 (+806 to +872 from transcription start site),
3. RPS23 exon 1,
4. first intron of RPS23 , and
5. nine bases of exon 2 RPS23 for splicing.
6. /
7. The ATG site in exon 1 was mutated to CTG.

### 7. RPS30I-M1TOD

### SEQ ID NO. 7

### Modifications

Same as RPS30I-M1 (SEQ ID NO. 3), namely
1. 5' truncated RPS30 promoter (+from transcriptional start site),
2. 60 bases were added that include sp1 site,
3. RPS30 exon 1,
4. first intron of RPS30 , and
5. nine bases of exon 2 RPS23 for splicing.
6. The putative transcriptional initiation box was improved from tacaaata to tataaata.
7. BamH1 site CACTGAG was eliminated by mutating to CACCTTGAG. in addition
8. includes tetO sequences (bold) for regulatable of transcription downstream of the TATAAA (underlined) signal.

RPS30-M1 has two sp1 sites: TCCCGCCCCGTCCTGCG and GGGGCGGAGC.

### 8. RPS30I-MITOU

### SEQ ID NO. 8

### Modifications

Same as RPS30I-M1 (SEQ ID NO. 3), namely
1. 5' truncated RPS30 promoter (+from transcriptional start site),
2. 60 bases were added that include sp 1 site,
3. RPS30 exon 1,
4. first intron of RPS30 , and
5. nine bases of exon 2 RPS23 for splicing.
6. The putative transcriptional initiation box was improved from tacaaata to tataaata.
7. BamH1 site CACTGAG was eliminated by mutating to CACCTTGAG. in addition
8. includes tetO sequences (bold) for regulatable of transcription upstream of the TATAAA (underlined) signal.

The following drawings and examples illustrate the present invention without, however, limiting the same thereto.

### Brief description of the drawings

**Figure 1****.**
   **A** Ribosomal Protein Promoter Reporter constructs.
      The promoters with their introns were amplified from genomic DNA using PCR with primers specific to the flanking region of each promoter/intron sequence. The primers included the restriction sites, EcoRV and SalI sites and the resultant PCR products were cloned onto EGFP expression vector that was previously cut by EcoRV and SalI. Ribosomal proteins promoters were amplified from genomic DNA of THP-1 cell line.
   **B** HEK293 cells in 96-well plates were transfected with 150 ng of EGFP reporter vectors that are under the transcriptional control of the ribosomal protein RPS30, RPS23I-M, or RPS30I-M1 sequences. The EGFP reporter contains control stable 3'UTR. After approximately 20 hr, the cells were treated with the following compounds: okadaic acid (100 nM) or TNF-α (10 ng/ml) to induce post-transcriptional induction, if any. High resolution images were obtained automatically by BD high-content imager. Quantitation was performed with our in-house ProXcell imaging algorithm.
**Figure 2****.**
   HEK293 cells in 96-well plates were transfected with 150 ng of EGFP reporter vectors that are under the transcriptional control of the CMV or SV40 promoter. After approximately 20 hr, the cells were treated with the following compounds: okadaic acid (100 nM) or TNF-α (10 ng/ml) to induce post-transcriptional induction, High resolution images were obtained automatically by BD high-content imager. Quantitation was performed with ProXcell imaging algorithm.
**Figure 3****.**
   Selective for post-transcriptional regulation in the RPS30I-M linked -reporter system.
   HEK293 cells in 96-well plates were transfected with 150 ng of EGFP reporter vectors that are under the transcriptional control of the ribosomal protein RPS30I-M1. The EGFP reporter contains either control stable 3'UTR or 3'UTR which contain 200 bases of IL-8 3'UTR sequences known to contain mRNA destabilization elements, AU-rich elements. After approximately 20 hr, the cells were treated with the following compounds: okadaic acid (100 nM) or IL-α (3 ng/ml) to induce post-transcriptional induction, if any. High resolution images were obtained automatically by BD high-content imager. Quantitation was performed with our in-house ProXcell imaging algorithm.
**Figure 4****.**
   Response of RPS promoter-linked reporter to ARE-3'UTR.
   HEK293 cells in 96-well plates were transfected with 150 ng of EGFP reporter vectors that are under the transcriptional control of the ribosomal protein RPS30I-M1 or CMV promoter. The EGFP reporter contains either control stable 3'UTR or 3'UTR which contain 200 bases of IL-8 or TNF-α 3'UTR sequences known to contain mRNA destabilization elements, AU-rich elements. High resolution images were obtained automatically by BD high-content imager. Quantitation was performed with our in-house ProXcell imaging algorithm.

### Examples

### Examples of RPS-linked performance

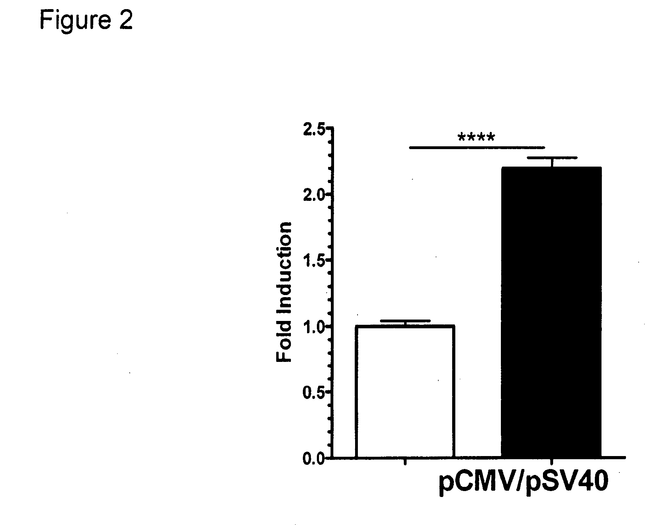

Figure 1A shows examples of the reporter constructs utilizing ribosomal protein promoters (RPS). The modification of RPS30I-M1 and RPS23I-M resulted in significant enhancement of the vector expression when compared to the expression vector under the control of the wild type RPS30 as evaluated by the fluorescence activity due to the EGFP reporter (Figure 1B). There was no induction by the inflammatory inducer, TNF-α, or the phosphatase inhibitor drug, okdadaic acid (Figure 1B). Unlike CMV and SV40 which tend to respond to transcriptional induction, for example, by TNF-α (Figure 2), the RPS30I-M1 has the advantage because assessment of changes at the post-transcriptional level can be examined without interference or minimal interference from transcriptional induction.

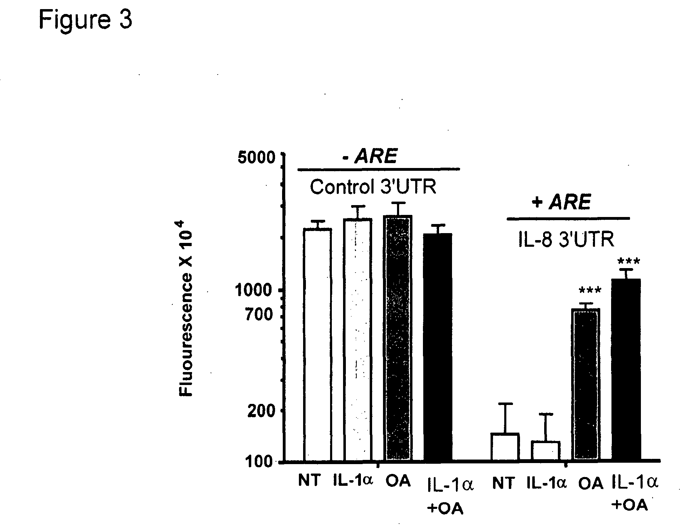

The RPS30I-M1 promoter (Figure 3) was chosen further for post-transcriptional assessment since it has both moderate constitutive expression and is not transcriptionally inducible by general stimulus, TNF-α or okadaic acid (Figure 1B). The AU-rich regions, approximately 200 bases, of TNF-α and IL-8 3'UTR were cloned into the vector. Briefly, a 237 bp region (972-1209 nt: NM_000584, SEQ ID NO. 9) that belongs to the 1250 bp IL-8 3'UTR or 250 bases from TNF-α 3'UTR: (1200-1450 bp, NM_000594, SEQ ID NO. 10) were amplified by RT-PCR using specific primers that contains BamH1 and XbaI restriction sites. The PCR products were cut by BamH I and Xba I sequentially and followed by phenol extraction and ethanol precipitation. The purified cut PCR products were ligated into the EGFP expression vector that has bovine growth hormone 3'UTR that has BamH1 and XbaI sites. Recombinant colonies were verified by PCR using a forward vector specific primer and IL-8 and TNF-α 3'UTRs reverse primer. Any cloning method known in the art can be used to clone AU-rich element regions.

The RPS30I-M1 with control 3'UTR, RPS30I-M1 with IL-8 3'UTR, and RPS30I-M1 with TNF-α 3'UTR vectors were tested for use to assess post-transcriptional regulation. Transfected cells were stimulated with the following compounds, IL-α or okadaic acid. Any compound or modulators such as protein, expression vector, or small inhibitory RNA, or any other RNA vector can be tested for the post-transcriptional effects, i.e., induction or repression. The okadaic acid which is phosphatase inhibitor significantly increases the RPS30I-M1-IL-8 3'UTR reporter and IL-α reporters but not control RPS30-3'UTR (Figure 3). This indicates that the use of non-inducible promoter of a ribosomal protein is useful to assess post-transcriptional effect.

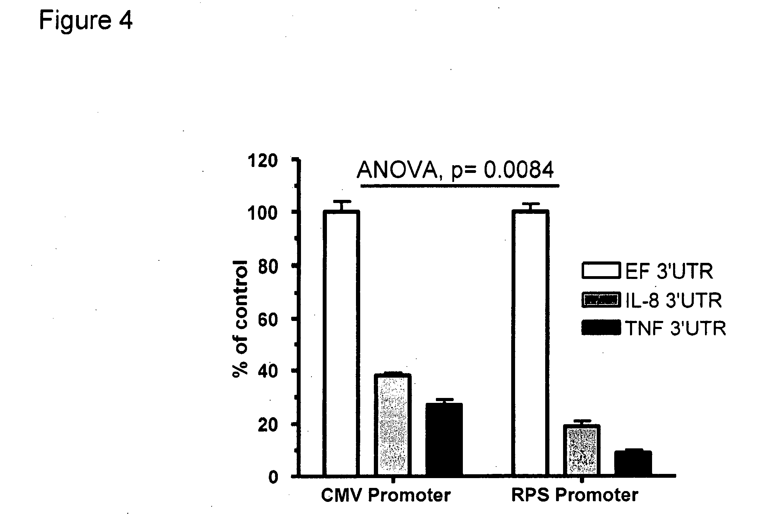

Moreover, responses to down-regulation due the presence of mRNA destabilization elements, AU-rich elements, are stronger with reporter constructs under the RPS30I-M1 promoter when compared to those with CMV promoter (Figure 4).

### References

Al-Zoghaibi, F., Ashour, T., Al-Ahmadi, W., Abulleef, H., Demirkaya, O., and Khabar, K.S. (2007). Bioinformatics and experimental derivation of an efficient hybrid 3' untranslated region and use in expression active linear DNA with minimum poly(A) region. Gene 391, 130-139.
Calzado, M.A., Bacher, S., and Schmitz, M.L. (2007). NF-kappaB inhibitors for the treatment of inflammatory diseases and cancer. Curr Med Chem 14, 367-376.
Cooper, S.J., Trinklein, N.D., Anton, E.D., Nguyen, L., and Myers, R.M. (2006). Comprehensive analysis of transcriptional promoter structure and function in 1% of the human genome. Genome Res. 16, 1-10.
Eisenberg, E. and Levanon, E.Y. (2003). Human housekeeping genes are compact. Trends Genet 19, 362-365.
Fong, C.L., Siddiqui, A.H., and Mark, D.F. (1994). Identification and characterization of a novel repressor site in the human tumor necrosis factor alpha gene. Nucleic Acids Res 22,1108-1114.
Gossen, M. and Bujard, H. (2002). Studying gene function in eukaryotes by conditional gene inactivation. Annu Rev Genet 36, 153-173.
Grandvaux, N., Servant, M.J., tenOever, B., Sen, G.C., Balachandran, S., Barber, G.N., Lin, R., and Hiscott, J. (2002). Transcriptional profiling of interferon regulatory factor 3 target genes: direct involvement in the regulation of interferon-stimulated genes. Journal of virology 76, 5532-5539.
Lai, C., Jiang, X., and Li, X. (2006). Development of luciferase reporter-based cell assays. Assay Drug Dev Technol 4, 307-315.
Li, X., Zhao, X., Fang, Y., Jiang, X., Duong, T., Fan, C., Huang, C.C., and Kain, S.R. (1998). Generation of destabilized green fluorescent protein as a transcription reporter. J Biol Chem 273, 34970-34975.
Khabar, K.S. and Young H.A. (2007). Post-transcriptional control of the interferon system. Biochimie 89, 761-9.
Meyer-Ficca, M.L., Meyer, R.G., Kaiser, H., Brack, A.R., Kandolf, R., and Küpper, J.-H. (2004). Comparative analysis of inducible expression systems in transient transfection studies. Anal Biochem 334 9-19.
Naylor, L.H. (1999). Reporter gene technology: the future looks bright. Biochem Pharmacol 58, 749-757.
Paun, A. and Pitha, P.M. (2007). The IRF family, revisited. Biochimie 89, 744-753.
Voon, D.C., Subrata, L.S., Baltic, S., Leu, M.P., Whiteway, J.M., Wong, A., Knight, S.A., Christiansen, F.T., and Daly, J.M. (2005). Use of mRNA- and protein-destabilizing elements to develop a highly responsive reporter system. Nucleic Acids Res 33, e27.
Xu, Z.L., Mizuguchi, H., Ishii-Watabe, A., Uchida, E., Mayumi, T., and Hayakawa, T. (2001). Optimization of transcriptional regulatory elements for constructing plasmid vectors. Gene 272, 149-156.
Yew, N.S., Wysokenski, D.M., Wang, K.X., Ziegler, R.J., Marshall, J., McNeilly, D., Cherry, M., Osburn, W., and Cheng, S.H. (1997). Optimization of plasmid vectors for high-level expression in lung epithelial cells. Hum Gene Ther 8, 575-584.
Zhao, S., Ooi, S.L., and Pardee, A.B. (1995). New primer strategy improves precision of differential display. Biotechniques 18, 842-846, 848, 850.

## Claims

1. Use of an expression vector comprising
(a) a promoter region comprising a non-inducible constitutively active ribosomal protein gene promoter,
(b) an operably linked reporter or gene sequence, and
(c) a 3' untranslated region (3' UTR)
for assessing post-transcriptional effects.

2. The use according to claim 1, wherein the non-inducible constitutively active ribosomal protein gene promoter of (a)
comprises the ribosomal protein gene promoter of ribosomal protein S23 (RPS23) or ribosomal protein S30 (RPS30), or parts thereof,
or is derived therefrom.

3. The use according to claim 2, wherein the RPS23 promoter or the RPS30 promoter or parts thereof or derivatives thereof
are modified for higher expression by modifying the transcriptional initiation sequence.

4. The use according to any of claims 1 to 3, wherein the promoter region (a) comprises at least one sp 1 site-containing sequence.

5. The use according to any of claims 1 to 4, wherein the promoter region (a) further comprises
- intron sequence(s) of ribosomal proteins, or parts thereof,
- exon sequence(s) of ribosomal proteins, or parts thereof,
- tetracycline operator (tetO) sequences, and/or
- modified sequences wherein the modification eliminates a restriction site.

6. The use according to any of claims 1 to 5, comprising a nucleic acid sequence of any of SEQ ID NOs. 3 to 8 or a sequence complementary thereto.

7. The use according to any of claims 1 to 5, wherein the 3' UTR of the expression vector (c) contains mRNA destabilization or stabilization elements derived from a 3' UTR of a cellular mRNA.

8. The use according to claim 7, wherein the mRNA destabilization or stabilization elements are selected from AU-rich elements, GU-rich elements, or U-rich sequences.

9. The use according to any of claims 1 to 8 for identifying compounds that affect post-transcriptional regulation of genes.

10. A method for identifying compounds that affect post-transcriptional regulation of reporter(s) or gene(s),
comprising the following steps
(1) providing
at least one expression vector as defined in any of claims 1 to 8,
at least one linear expression cassette derived from the expression vector as defined in any of claims 1 to 8,
an array comprising at least two expression vectors as defined in any of claims 1 to 8 in one or more vessels,
wherein said at least two expression vectors differ in their 3'UTR or post-transcriptional control element(s),
a library of expression vectors comprising at least two expression vectors as defined in any of claims 1 to 8,
wherein each expression vector comprises a different 3'UTR or post-transcriptional control element(s), and/or
a cell library wherein each cell line of the cell library harbours an expression vector as defined in any of claims 1 to 8, wherein said expression vector comprises a 3'UTR or post-transcriptional control element(s) different from that of the expression vectors of the other cell lines,
(2) providing at least a compound to be tested,
(3) determining the effect of the compound on the post-transcriptional regulation by determining the mRNA level and/or the expression level of the reporter or gene.

11. The use according to claim 2, wherein the ribosomal protein gene promoter of ribosomal protein S23 (RPS23) or ribosomal protein S30 (RPS30) is the human RPS23 promoter or the human RPS30 promoter.

12. The use according to claim 3, wherein modifying the transcriptional initiation sequence occurs by mutating a TATA-like sequence to the TATA-signal sequence.

13. The use according to any of claims 3 and 12, wherein modifying the transcriptional initiation sequence occurs by substituting the TATA-like signal TACAAATA with the TATA signal TATAAATA.

14. The use according to claim 4, wherein the at least one sp1 site-containing sequence is obtained by truncating the RPS23 promoter or the RPS30 promoter and adding at least one sp1 site-containing sequence.

15. The use according to claim 5, wherein the ribosomal proteins are RPS30 and/or RPS 23.

## Patentansprüche

1. Verwendung eines Expressionsvektors, umfassend
(a) eine Promoterregion umfassend einen nicht induzierbaren konstitutiv aktiven ribosomalen Proteingenpromoter,
(b) eine funktionsbereit verbundene Reporter- oder Gensequenz, und
(c) eine 3' untranslatierte Region (3' UTR)
für die Beurteilung von post-transkriptionalen Effekten.

2. Verwendung gemäß Anspruch 1, wobei der nicht induzierbare konstitutiv aktive ribosomale Proteingenpromoter von (a)
den ribosomalen Proteingenpromoter des ribosomalen Proteins S23 (RPS23) oder des ribosomalen Proteins S30 (RPS30), oder Teile davon, umfasst oder davon abgeleitet ist.

3. Verwendung gemäß Anspruch 2, wobei der RPS23 Promoter oder der RPS30 Promoter oder Teile davon oder Derivative davon durch Modifizieren der transkriptionalen Startsequenz für höhere Expression modifiziert ist/sind.

4. Verwendung gemäß einem der Ansprüche 1 bis 3, wobei die Promoterregion (a) mindestens eine sp1 Stelle-enthaltende Sequenz umfasst.

5. Verwendung gemäß einem der Ansprüche 1 bis 4, wobei die Promoterregion (a) weiterhin umfasst
- Intronsequenz(en) von ribosomalen Proteinen, oder Teile davon,
- Exonsequenz(en) von ribosomalen Proteinen, oder Teile davon,
- Tetracyclin-Operator (tetO)-Sequenzen, und/oder
- modifizierte Sequenzen, wobei die Modifikation eine Restriktionsstelle eliminiert.

6. Verwendung gemäß einem der Ansprüche 1 bis 5, umfassend eine Nukleinsäuresequenz von einer von SEQ ID NOs. 3 bis 8 oder eine komplementäre Sequenz dazu.

7. Verwendung gemäß einem der Ansprüche 1 bis 5, wobei die 3' UTR des Expressionsvektors (c) mRNA Destabilisierungs- oder Stabilisierungselemente enthält, die von einer 3' UTR einer zellulären mRNA abgeleitet sind.

8. Verwendung gemäß Anspruch 7, wobei die mRNA Destabilisierungs- oder Stabilisierungselemente ausgewählt sind aus AU-reichen Elementen, GU-reichen Elementen oder U-reichen Sequenzen.

9. Verwendung gemäß einem der Ansprüche 1 bis 8 für die Identifizierung von Verbindungen, die die post-transkriptionale Regulation von Genen beeinflussen.

10. Verfahren für die Identifizierung von Verbindungen, die die post-transkriptionale Regulation von Reporter(n) und Gen(en) beeinflussen,
umfassend die folgenden Schritte
(1) zur Verfügung stellen von
mindestens einem Expressionsvektor, wie in einem der Ansprüche 1 bis 8 definiert, mindestens einer linearen Expressionskassette abgeleitet von dem Expressionsvektor, wie in einem der Ansprüche 1 bis 8 definiert,
einem Array umfassend mindestens zwei Expressionsvektoren, wie in einem der Ansprüche 1 bis 8 definiert, in einem oder mehreren Gefäßen,
wobei sich die mindestens zwei Expressionsvektoren in ihren 3'UTR oder post-transkriptionalen Kontrollelement(en) unterscheiden,
einer Bibliothek von Expressionsvektoren umfassend mindestens zwei Expressionsvektoren, wie in einem der Ansprüche 1 bis 8 definiert,
wobei jeder Expressionsvektor eine unterschiedliche 3'UTR oder unterschiedliche post-transkriptionale Kontrollelement(e) umfasst, und/oder
einer Zellbibliothek, wobei jede Zelllinie der Zellbibliothek einen Expressionsvektor, wie in einem der Ansprüche 1 bis 8 definiert, enthält, wobei der Expressionsvektor eine 3'UTR oder post-transkriptionale Kontrollelement(e) umfasst, die verschieden sind von denen der Expressionsvektoren der anderen Zelllinien,
(2) zur Verfügung stellen mindestens einer zu testenden Verbindung,
(3) Bestimmen des Effekts der Verbindung auf die the post-transkriptionale Regulation durch Bestimmen der mRNA-Level und/oder der Expression-Level des Reporters oder des Gens.

11. Verwendung gemäß Anspruch 2, wobei der ribosomale Proteingenpromoter des ribosomalen Proteins S23 (RPS23) oder des ribosomalen Proteins S30 (RPS30) der humane RPS23 Promoter oder der humane RPS30 Promoter ist.

12. Verwendung gemäß Anspruch 3, wobei das Modifizieren der transkriptionalen Startsequenz durch Mutation einer TATA-ähnlichen Sequenz in die TATA-Signalsequenz erfolgt.

13. Verwendung gemäß einem der Ansprüche 3 und 12, wobei das Modifizieren der transkriptionalen Startsequenz durch Substitution des TATA-ähnlichen Signals TACAAATA mit der TATA-Signalsequenz TATAAATA erfolgt.

14. Verwendung gemäß Anspruch 4, wobei die mindestens eine sp1 Stelle-enthaltende Sequenz durch Trunkieren des RPS23 Promoters oder des RPS30 Promoters und durch Addition mindestens einer sp1 Stelle-enthaltenden Sequenz erhalten wird.

15. Verwendung gemäß Anspruch 5, wobei die ribosomalen Proteine RPS30 und/oder RPS 23 sind.

## Revendications

1. Utilisation d'un vecteur d'expression comprenant
(a) une région promoteur comprenant un promoteur d'un gène de protéine ribosomique constitutivement active non inductible,
(b) une séquence de reporter ou de gène liée fonctionnellement, et
(c) une région non traduite en 3' (3' UTR)
pour évaluer des effets post-transcriptionnels.

2. Utilisation selon la revendication 1, dans laquelle le promoteur du gène de la protéine ribosomique constitutivement active non inductible de (a)
comprend le promoteur du gène de la protéine ribosomique S23 (RPS23) ou de la protéine ribosomique S30 (RPS30) ou des parties de celles-ci,
ou est dérivé de ceux-ci.

3. Utilisation selon la revendication 2, dans laquelle le promoteur RPS23 ou le promoteur RPS30 ou des parties de ceux-ci ou des dérivés de ceux-ci sont modifiés pour une expression supérieure en modifiant la séquence d'initiation transcriptionnelle.

4. Utilisation selon l'une quelconque des revendications 1 à 3, dans laquelle la région du promoteur (a) comprend au moins une séquence contenant un site sp1.

5. Utilisation selon l'une quelconque des revendications 1 à 4, dans laquelle la région promoteur (a) comprend en outre
- une/des séquence(s) d'intron de protéines ribosomiques ou de parties de celles-ci,
- une/des séquence(s) d'exon de protéines ribosomiques ou de parties de celles-ci,
- des séquences d'opérateur de tétracycline (tetO), et/ou
- des séquences modifiées dans lesquelles la modification élimine un site de restriction.

6. Utilisation selon l'une quelconque des revendications 1 à 5, comprenant une séquence d'acide nucléique selon l'une quelconque des SEQ ID N° : 3 à 8 ou une séquence complémentaire de celles-ci.

7. Utilisation selon l'une quelconque des revendications 1 à 5, dans laquelle la 3' UTR du vecteur d'expression (c) contient des éléments de déstabilisation ou de stabilisation de l'ARNm dérivés d'une 3' UTR d'un ARNm cellulaire.

8. Utilisation selon la revendication 7, dans laquelle les éléments de déstabilisation ou de stabilisation de l'ARNm sont choisis parmi les éléments riches en AU, les éléments riches en GU ou les séquences riches en U.

9. Utilisation selon l'une quelconque des revendications 1 à 8, pour identifier des composés qui affectent la régulation post-transcriptionnelle des gènes.

10. Procédé d'identification de composés qui affectent la régulation post-transcriptionnelle du/des reporter(s) ou gène(s),
comprenant les étapes suivantes
(1) la fourniture
d'au moins un vecteur d'expression tel que défini dans l'une quelconque des revendications 1 à 8,
d'au moins une cassette d'expression linéaire dérivée du vecteur d'expression tel que défini dans l'une quelconque des revendications 1 à 8,
d'une puce comprenant au moins deux vecteurs d'expression tels que définis selon l'une quelconque des revendications 1 à 8, dans un ou plusieurs récipients,
dans laquelle lesdits au moins deux vecteurs d'expression diffèrent dans leur 3' UTR ou leur(s) élément(s) de contrôle post-transcriptionnels,
d'une banque de vecteurs d'expression comprenant au moins deux vecteurs d'expression tels que définis dans l'une quelconque des revendications 1 à 8,
dans laquelle chaque vecteur d'expression comprend une 3' UTR ou un/des élément (s) de contrôle post-transcriptionnels différents, et/ou
d'une banque de cellules dans laquelle chaque lignée cellulaire abrite un vecteur d'expression tel que défini dans l'une quelconque des revendications 1 à 8, dans laquelle le vecteur d'expression comprend une 3' UTR ou un/des élément(s) de contrôle post-transcriptionnels différents de ceux des vecteurs d'expression des autres lignées cellulaires,
(2) la fourniture d'au moins un composé à tester,
(3) la détermination de l'effet du composé sur la régulation post-transcriptionnelle en déterminant le niveau d'ARNm et/ou le niveau d'expression du reporter ou du gène.

11. Utilisation selon la revendication 2, dans laquelle le promoteur du gène de la protéine ribosomique S23 (RPS23) ou de la protéine ribosomique S30 (RPS30) est le promoteur RPS23 humain ou le promoteur RPS30 humain.

12. Utilisation selon la revendication 3, dans laquelle la modification de la séquence d'initiation transcriptionnelle se produit par mutation d'une séquence de type TATA en séquence de signal TATA.

13. Utilisation selon l'une quelconque des revendications 3 et 12, dans laquelle la modification de la séquence d'initiation transcriptionnelle se produit en substituant le signal de type TATA, TACAAATA par le signal TATA, TATAAATA.

14. Utilisation selon la revendication 4, dans laquelle l'au moins une séquence contenant le site sp1 est obtenue par troncature du promoteur RPS23 ou du promoteur RPS30 et en ajoutant au moins une séquence contenant un site sp1.

15. Utilisation selon la revendication 5, dans laquelle les protéines ribosomiques sont RPS30 et/ou RPS23.


### SEQUENCE LISTING

<110> King Faisal Specialist Hospital & Research Centre
<120> Expression Vectors based on modified ribosomal protein promoters and uses thereof in post-transcriptional assessment
<130> K30346PCT
<160> 10
<170> PatentIn version 3.3
<210> 1
   <211> 3504
   <212> DNA
   <213> Homo sapiens
<400> 1
   agcgtggcct tgtttgtacc tccatgattg cctggctggc cttgctaacc taatcacatc    60
   tgtgacggga tatagtgatg tttaatctta tgattgcctt aagaattaag gcaatcagac   120
   gggttcggcg gctcatgcct gtaatcccag cactttggga ggccgaggcg ggcggatcac   180
   gaggtcagaa gatccagtcc atcctggcta acaaggtgaa accccgtctc tactaaaaat   240
   acaaaaaatt agccgggcat ggtggcggga gcctgtagtc ccagctactc gggaggctga   300
   ggcaggagga tggcgtgaat ctgggaggcg gagcttgcag tgggccgaga tcgcgccact   360
   gccctccagc ctgggcgaca gagcgagact ccgtctcaaa aaaaaaaaaa aaaagaatta   420
   aggcaatcat aattccccac gcacactcat atgctaggac cccgcccctt acctgaaacg   480
   ttgtggctta tatagacact gccaggcact gtgttaagtg ctcccaaaga gcaccccagt   540
   ctaccatttt ccctctcgat tctatatgta cactcgggac aagttctcct gatcgaaaac   600
   ggcaaaacta aggccccaag taggaatgcc ttagttttcg gggttaacaa tgattaacac   660
   tgagcctcac acccacgcga tgccctcagc tcctcgctca gcgctctcac caacagccgt   720
   agcccgcagc cccgctggac accggttctc catccccgca gcgtagcccg gaacatggta   780
   gctgccatct ttacctgcta cgccagcctt ctgtgcgcgc aactgtctgg tcccgccccg   840
   tcctgcgcga gctgcctgcc caggcaggtt cgccggtgcg agcgtaaagg ggcggagcta   900
   ggactgcctt gggcggtaca aatagcaggg aaccgcgcgg tcgctcagca gtgacgtgac   960
   acgcagccca cggtctgtac tgacgcgccc tcgcttcttc ctctttctcg actccatctt  1020
   cgcggtagct gggaccgccg ttcaggtaag aatggggcct tggctggatc cgaagggctt  1080
   gtagcaggtt ggctgcgggg tcagaaggcg cggggggaac cgaagaacgg ggcctgctcc  1140
   gtggccctgc tccagtccct atccgaactc cttgggaggc ctggccttcc ccacgtgagc  1200
   cgccgcgacc accatcccgt cgcgatcgtt tctggaccgc tttccactcc caaatctcct  1260
   ttatcccaga gcatttcttg gcttctctta caagccgtct tttctttact cagtcgccaa  1320
   tatgcagctc tttgtccgcg cccaggagct acacaccttc gaggtgaccg gccaggaaac 1380
   ggtcgcccag atcaaggtaa ggctgcttgg tgcgccctgg gttccatttt cttgtgctct 1440
   tcactctcgc ggcccgaggg aacgcttacg agccttatct ttccctgtag gctcatgtag 1500
   cctcactgga gggcattgcc ccggaagatc aagtcgtgct cctggcaggc gcgcccctgg 1560
   aggatgaggc cactctgggc cagtgcgggg tggaggccct gactaccctg gaagtagcag 1620
   gccgcatgct tggaggtgag tgagagagga atgttctttg aagtaccggt aagcgtctag 1680
   tgagtgtggg gtgcatagtc ctgacagctg agtgtcacac ctatggtaat agagtacttc 1740
   tcactgtctt cagttcagag tgattcttcc tgtttacatc cctcatgttg aacacagacg 1800
   tccatgggag actgagccag agtgtagttg tatttcagtc acatcacgag atcctagtct 1860
   ggttatcagc ttccacacta aaattaggtc agaccagggc ccccaaagtg ctctataaaa 1920
   ttagaagctg gaagatcctg aaatgaaact taagatttca aggtcaaata tctgcaactt 1980
   tgttctcatt acctattggg cgcagcttct ctttaaaggc ttgaattgag aaaagagggg 2040
   ttctgctggg tggcaccttc ttgctcttac ctgctggtgc cttcctttcc cactacaggt 2100
   aaagtccatg gttccctggc ccgtgctgga aaagtgagag gtcagactcc taaggtgagt 2160
   gagagtatta gtggtcatgg tgttaggact ttttttcctt tcacagctaa accaagtccc 2220
   tgggctctta ctcggtttgc cttctccctc cctggagatg agcctgaggg aagggatgct 2280
   aggtgtggaa gacaggaacc agggcctgat taaccttccc ttctccaggt ggccaaacag 2340
   gagaagaaga agaagaagac aggtcgggct aagcggcgga tgcagtacaa ccggcgcttt 2400
   gtcaacgttg tgcccacctt tggcaagaag aagggcccca atgccaactc ttaagtcttt 2460
   tgtaattctg gctttctcta ataaaaaagc cacttagttc agtcatcgca ttgtttcatc 2520
   tttacttgca aggcctcagg gagaggtgtg cttctcgggt tggtggtatg tcccctagga 2580
   gaacagtgag gcagaaaagg cagaagcctt tggtatgggg ggaagaaatg gtaaactaca 2640
   agagaaattt cctgtgaaga aacagctaca gatcctgggg ggcttcagat gtaaaattgg 2700
   ggttattccc tatcctaagt aacttgatca gtccccccag gtcattcttt ttcatcttct 2760
   aaacagagaa ggtagcagga atcactgtgg tgagaggttt gttatggagg cagcaataga 2820
   agggatgggt gggggaagag gtttgtatag aaggtgaacc tggccgttcc ctgaacttgg 2880
   taccagctgt ggccttagag tccagggcag gaatctggtc tgccttggtt ttagaagtaa 2940
   atattatgtt gggagcatgg cctcgtttgt acctctgtga ctgcctggcc ggacttggta 3000
   acctaatcac atctgtgatt ggatatagtg aggtttcagt gttcccaaaa gttgggttac 3060
   ctctggggct gattcagggt tctcttctgg caactgagcc tcccagcact tctgaacccc 3120
   acttactcat tcagctaaag tttctggacc tgccagttct tgagaaatag catccaacag 3180
   ggtaaagccc ttgggctgtg gactttgact gcctgagttt ggaccttctt tttcttccta 3240
   ctccatttac tgggtggctg gcctttgaac taactactaa tttaatctct gccatctcca   3300
   gggctgctgt gagggttaaa ggatgtaaat caacatctgg cttacagtga gtgtgtgaat   3360
   cttggctatt tttgtctctg tggtgttaaa gacatggttt ctgccttcca gcagtttaga   3420
   aagggggagg atgtggacag atacaatagc atcccagaga gggcctcttt ttttgttttt   3480
   cttttttctt tattttattt tatt                                          3504
<210> 2
   <211> 4289
   <212> DNA
   <213> Homo sapiens
<400> 2
   gtctggcaca tagaaggcat ttttaaacat ccttgctgag tgaaccaata tcccagaaac 60
   ctctcacagc tagttcatct tacaggagaa acagtattaa agagttaatt aaatggccag 120
   gcgcggtggc tcacgcctgt aatcccagca cattgggagg ccgaggcggg cggatcactc 180
   gaggtcagga gatcgacacc agcctggcca acatggtgaa accccctctc tactgaaaat 240
   acaaacatca gccaggcgtg atggtggaag cctgtaatcc cagctactca ggaggctgag 300
   gtgggacaat cgcttgaacc cgggaggcgg aggttgtagt gagccaagat cgcaccactg 360
   cacaacagcc tagaagacag agtgagaccc tgtctcagaa aaaaataaaa ataaaaataa 420
   ataaatctat aagtaaatga ctcgccagtc aaaataaacg gcaactttag ggttaaaggc 480
   ccaatctggc tccaaagctt ggggttttag ttactacact acattgcttc actatatttt 540
   acaatttact agctgcttat aagtatgaat taaggctcag aagtctaatt ttccagacta 600
   ctcggaggac tctcgcccca ctccactcca caaagattca gctcagcgac tccttcctac 660
   tctgacctag ccccgcgtcc cgctctcagt ggcttgggca agagcgcctg cgcggtgagc 720
   gggtcccata aaacgcattc tgggattggt agtccatgtt cctccggtct ccagcattca 780
   aaagaaaaag ggggaaaaaa aaccatgcaa attagatatc tctgaatttc ttgcaaatta 840
   aataagacgc agattctggc tcaggaaagt gatgcaaacg cgtcgttttc aaaggagaga 900
   ccccagcctc gggtcaggcg cggcgcagac agcggcgcgg ggtccttggc tgggcggggc 960
   ttgctcgcgg tggcttgtgg ctccttcctg cggtgcttct ctctttcgct caggcccgtg 1020
   gcgccgacag gatgggtgag ctgttgtggc cggtttaagg gcgctgcaag cgggacttgg 1080
   ggtcttgggg acgggcgggc ggatgcgaat agagtagggc gggggatgcc atggagaggc 1140
   tccatggggg agggccgggg aagcgccgct ccaggaggca cgtggtccgg cgcggaaggg 1200
   gcccatgagg cgtggaggcc gccgaggtcg gggtaccgag ggacgcaggg aggccagcgc 1260
   ttcctcccgg gcattcgagc ggggcctcgt ccttcgggag aacacattct ccggagccct 1320
   cttcgaacgt ttattagtcg gttcagggca acttgaaggc caaatgtttg gcccacaggc 1380
   caataaatag tacgagagcc aatcggctta agggtttatt ccaggtgagg cgagtgtctt 1440
   agaagatggg aaacacgtag atggcgtgtt tttacggaag aactaaaata tttaattttt 1500
   aggcaagtgt cgtggacttc gtactgctag gaagctccgt agtcaccgac gagaccagaa 1560
   gtggcatgat aaacagtata agaaagctca tttgggcaca gccctaaagg ccaacccttt 1620
   tggaggtgct tctcatgcaa aaggaatcgt gctggaaaaa gtgtaagtcc attgctcccg 1680
   tcaagtttta gtttattata ggaattcgag acatgaactt acgaattctt gttttgaaag 1740
   taattgcagg tttttgtgta gtagtattca tttgggcatt gtggggtaaa attgcaaagc 1800
   gtttgttcta tttaaaagtt ggtaaaatta gtttttggga attaggtagt taaggtttta 1860
   atttaacgtt ggcctggaag gaattggaga agatactagc aatgatgaag taaaggacac 1920
   aaacaccttt actgtgggag ttgttataag taaatggcac gtgtcagcta ttgaacttta 1980
   tcgacttgat aaaactaagg tgaagagaag tgacttgcat cagaattaat tgaggtcata 2040
   cacctaagat tgagacatga aactgccagt atttgactgg ttttgacttt ttaaaataat 2100
   aatttcatat agttctatca tatttgatgg tagagccatt ttaacccaga cttttttttt 2160
   tttttttttt ttttttgaga cagtctagct ctgtcaccca ggctggtgtg cagtagcgca 2220
   agactccctg caaccttagc ctcccaggtt caagcatttc tcctgcctca gcctcccagg 2280
   tagctgggat tacaggcgcc cactaccaca ccagctaata ttttgtattt tcagtagtga 2340
   tggggtttca ccatgttgac caggctagtc tcaaactcct gacctcaggt gataatgcct 2400
   gcttcggcct ccgaaagtgc tggaattaca ggcgtgagcc actgtgtccg gcccagactt 2460
   tctaattctt acctcagata ccttttttct tttttctttt tttttttttg agatagggtc 2520
   ccttgtcaca caggctggcc atcttgacgt tctaggcata gatcctccca cgtcagcctc 2580
   gcaagtagtt gggactacag gcccacgctg ccactccagt ctacttttat aactgtaaaa 2640
   ggtctagaaa tttcccccat tgtgctaatg aaattaagac tggcagaaaa ctaggttgac 2700
   atcacaggac ttcagctcag ccatttgagg ttagattgaa aagatagaaa cagtttctca 2760
   ttagttctct agttaatatg aaaagataat ctttttcaga aagccagctc acagtgctgt 2820
   gccttttgta tttcagagga gttgaagcca aacagccaaa ttctgccatt aggaagtgtg 2880
   taagggtcca gctgatcaag aatggcaaga aaatcacagc ctttgtaccc aatgacggtt 2940
   gcttgaactt tattgaggtg agtatttcaa ctctatcgta ccttctgttc ttggggtggc 3000
   ctccctcaca tttttatctg atgcaaggga gtttcctcac atgaaagtat ttttgtgatc 3060
   gccaccaaca ccagaaataa acttcttatt ttattccagg aaaatgatga agttctggtt 3120
   gctggatttg gtcgcaaagg tcatgctgtt ggtgatattc ctggagtccg ctttaaggtt 3180
   gtcaaagtag ccaatgtttc tcttttggcc ctatacaaag gcaagaagga aagaccaaga 3240
   tcataaatat taatggtgaa aacactgtag taataaattt tcatatgcca aaaaatgttt   3300
   gtatcttact gtcccctgtt ctcaccacga agatcatgtt cattaccacc accacccccc    3360
   cttatttttt ttatcctaaa ccagcaaacg caggacctgt accaatttta ggagacaata   3420
   agacagggtt gtttcaggat tctctagagt taataacatt tgtaacctgg cacagtttcc   3480
   ctcatcctgt ggaataagaa aatgggatag atctggaata aatgtgcagt attgtagtat   3540
   tactttaaga actttaaggg aacttcaaaa actcactgaa attctagtga gatactttct   3600
   tttttattct tggtattttc catatcgggt gcaacacttc agttaccaaa tttcattgca   3660
   catagattat cttaggtacc cttggaaatg cacattcttg tatccatctt acaggggccc   3720
   aagatgataa atagtaaact caaaattgct ccccactctg tttattattt aaaggtgtca   3780
   ggatctgtgt tgtaatgtgt ctacattaat gtgtttagga gaatacaggc attggatcat   3840
   ttagttgatg gaagtatatg ccaggcaagg gagataaggt atacgacaag actgatgttt   3900
   tcagtatctt ctcatgaggt tgtcagagac cttcatgtct tcaaagacta gtcagcaaat   3960
   gaagtggttt agtgtagaga caagattggt tgtgttttga taatttaagc taggtattga   4020
   gtacatgtgg attttgctgt ccacaaatac ttgtttcaga gttttcatgg atacagtggc   4080
   atggttgaaa tgaagctgtg agccttctgc tttaaatctg atgtaagaaa ctcctgttaa   4140
   caaatagtaa gtatgggtta attagccctt tgatcaaagc ctagctttac attgtttagg   4200
   atctttggaa aacaattggt ttggttgccc actttccgta ggatcaagag cagaaccttt   4260
   cacatggcac agaagaaccc aggttgcgc                                     4289
<210> 3
   <211> 733
   <212> DNA
   <213> Artificial
<220>
   <223> modified RPS30 promoter region
<400> 3
   gatatcggca aaactaaggc cccaagtagg aatgccttag ttttcggggt taacaatgat     60
   taacactcct gagcctcaca cccacgcgat gccctcagct cctcgctcag cgctctcacc    120
   aacagccgta gcccgcagcc ccgctggaca ccgggtctcc atccccgcag cgtagcccgg    180
   aacatggtag ctgccatctt tacctgctac gccagccttc tgtgcgcgca actgtctggt    240
   cccgccccgt cctgcgcgag ctgcctgccc aggcaggttc gccggtgcga gcgtaaaggg    300
   gcggagctag gactgccttg ggcggtataa atagcaggga accgcgcggt cgctcagcag    360
   tgacgtgaca cgcagcccac ggtctgtact gacgcgccct cgcttcttcc tctttctcga    420
   ctccatcttc gcggtagctg ggaccgccgt tcaggtaaga atggggcctt ggctgcagcc    480
   gaagggcttg tagcaggttg gctgcggggt cagaaggcgc ggggggaacc gaagaacggg    540
   gcctgctccg tggccctgct ccagtcccta tccgaactcc ttgggaggcc tggccttccc    600
   cacgtgagcc gccgcgacca ccatcccgtc gcgatcgttt ctggaccgct ttccactccc    660
   aaatctcctt tatcccagag catttcttgg cttctcttac aagccgtctt ttctttactc    720
   agtcgccgtc gac                                                       733
<210> 4
   <211> 781
   <212> DNA
   <213> Artificial
<220>
   <223> modified RPS30 promoter region
<400> 4
   gatatctagc cgggcatggt ggcgggagcc tgtagtccca gctactcggg aggctgaggc 60
   aggaggatgg cgtgaatctg ggaggcggag cttgcagtgg gccgagatcg cgccacttga 120
   gcctcacacc cacgcgatgc cctcagctcc tcgctcagcg ctctcaccaa cagccgtagc 180
   ccgcagcccc gctggacacc gggtctccat ccccgcagcg tagcccggaa catggtagct 240
   gccatcttta cctgctacgc cagccttctg tgcgcgcaac tgtctggtcc cgccccgtcc 300
   tgcgcgagct gcctgcccag gcaggttcgc cggtgcgagc gtaaaggggc ggagctagga 360
   ctgccttggg cggtacaaat agcagggaac cgcgcggtcg ctcagcagtg acgtgacacg 420
   cagcccacgg tctgtactga cgcgccctcg cttcttcctc tttctcgact ccatcttcgc 480
   ggtagctggg accgccgttc aggtaagaat ggggccttgg ctgcagccga agggcttgta 540
   gcaggttggc tgcggggtca gaaggcgcgg ggggaaccga agaacggggc ctgctccgtg 600
   gccctgctcc agtccctatc cgaactcctt gggaggcctg gccttcccca cgtgagccgc 660
   cgcgaccacc atcccgtcgc gatcgtttct ggaccgcttt ccactcccaa atctccttta 720
   tcccagagca tttcttggct tctcttacaa gccgtctttt ctttactcag tcgccgtcga 780
   c 781
<210> 5
   <211> 781
   <212> DNA
   <213> Artificial
<220>
   <223> modified RPS30 promoter region
<400> 5
   gatatctagc cgggcatggt ggcgggagcc tgtagtccca gctactcggg aggctgaggc 60
   aggaggatgg cgtgaatctg ggaggcggag cttgcagtgg gccgagatcg cgccacttga 120
   gcctcacacc cacgcgatgc cctcagctcc tcgctcagcg ctctcaccaa cagccgtagc 180
   ccgcagcccc gctggacacc gggtctccat ccccgcagcg tagcccggaa catggtagct 240
   gccatcttta cctgctacgc cagccttctg tgcgcgcaac tgtctggtcc cgccccgtcc 300
   tgcgcgagct gcctgcccag gcaggttcgc cggtgcgagc gtaaaggggc ggagctagga 360
   ctgccttggg cggtataaat agcagggaac cgcgcggtcg ctcagcagtg acgtgacacg 420
   cagcccacgg tctgtactga cgcgccctcg cttcttcctc tttctcgact ccatcttcgc 480
   ggtagctggg accgccgttc aggtaagaat ggggccttgg ctgcagccga agggcttgta 540
   gcaggttggc tgcggggtca gaaggcgcgg ggggaaccga agaacggggc ctgctccgtg 600
   gccctgctcc agtccctatc cgaactcctt gggaggcctg gccttcccca cgtgagccgc 660
   cgcgaccacc atcccgtcgc gatcgtttct ggaccgcttt ccactcccaa atctccttta 720
   tcccagagca tttcttggct tctcttacaa gccgtctttt ctttactcag tcgccgtcga 780
   c 781
<210> 6
   <211> 992
   <212> DNA
   <213> Artificial
<220>
   <223> modified RPS23 promoter region
<400> 6
   gatatctggc caggcgcggt ggctcacgcc tgtaatccca gcacattggg aggccgaggc 60
   gggcggatca ctcccagact actcggagga ctctcgcccc actccactcc acaaagattc 120
   agctcagcga ctccttccta ctctgaccta gccccgcgtc ccgctctcag tggcttgggc 180
   aagagcgcct gcgcggtgag cgggtcccat aaaacgcatt ctgggattgg tagtccatgt 240
   tcctccggtc tccagcattc aaaagaaaaa gggggaaaaa aaaccatgca aattagaatc 300
   tctgaatttc ttgcaaatta aataagacgc agattctggc tcaggaaagt gatgcaaacg 360
   cgtcgttttc aaaggagaga ccccagcctc gggtcaggcg cggcgcagac agcggcgcgg 420
   ggtccttggc tgggcggggc ttgctcgcgg tggcttgtgg ctccttcctg cggtgcttct 480
   ctctttcgct caggcccgtg gcgccgacag gctgggtgag ctgttgtggc cggtttaagg 540
   gcgctgcaag cgggacttgg ggtcttgggg acgggcgggc ggatgcgaat agagtagggc 600
   gggggatgcc atggagaggc tccatggggg agggccgggg aagcgccgct ccaggaggca 660
   cgtggtccgg cgcggaaggg gcccatgagg cgtggaggcc gccgaggtcg gggtaccgag 720
   ggacgcaggg aggccagcgc ttcctcccgg gcattcgagc ggggcctcgt ccttcgggag 780
   aacacattct ccggagccct cttcgaacgt ttattagtcg gttcagggca acttgaaggc 840
   caaatgtttg gcccacaggc caataaatag tacgagagcc aatcggctaa gggtttattc 900
   caggtgaggc gagtgtctta gaagatggga aacacgtaga tggcgtgttt ttacggaaga 960
   actaaaatat ttaattttta ggcaaggtcg ac 992
<210> 7
   <211> 793
   <212> DNA
   <213> Artificial
<220>
   <223> modified RPS30 promoter region
<400> 7
   gatatcggca aaactaaggc cccaagtagg aatgccttag ttttcggggt taacaatgat 60
   taacactcct gagcctcaca cccacgcgat gccctcagct cctcgctcag cgctctcacc 120
   aacagccgta gcccgcagcc ccgctggaca ccgggtctcc atccccgcag cgtagcccgg 180
   aacatggtag ctgccatctt tacctgctac gccagccttc tgtgcgcgca actgtctggt 240
   cccgccccgt cctgcgcgag ctgcctgccc aggcaggttc gccggtgcga gcgtaaaggg 300
   gcggagctag gactgccttg ggcggtataa atagcaggga catccctatc agtgatagac 360
   catccctatc agtgatagac catccctatc agtgatagac accgcgcggt cgctcagcag 420
   tgacgtgaca cgcagcccac ggtctgtact gacgcgccct cgcttcttcc tctttctcga 480
   ctccatcttc gcggtagctg ggaccgccgt tcaggtaaga atggggcctt ggctgcagcc 540
   gaagggcttg tagcaggttg gctgcggggt cagaaggcgc ggggggaacc gaagaacggg 600
   gcctgctccg tggccctgct ccagtcccta tccgaactcc ttgggaggcc tggccttccc 660
   cacgtgagcc gccgcgacca ccatcccgtc gcgatcgttt ctggaccgct ttccactccc 720
   aaatctcctt tatcccagag catttcttgg cttctcttac aagccgtctt ttctttactc 780
   agtcgccgtc gac 793
<210> 8
   <211> 793
   <212> DNA
   <213> Artificial
<220>
   <223> modified RPS30 promoter region
<400> 8
   gatatcggca aaactaaggc cccaagtagg aatgccttag ttttcggggt taacaatgat 60
   taacactcct gagcctcaca cccacgcgat gccctcagct cctcgctcag cgctctcacc 120
   aacagccgta gcccgcagcc ccgctggaca ccgggtctcc atccccgcag cgtagcccgg 180
   aacatggtag ctgccatctt tacctgctac gccagccttc tgtgcgcgca actgtctggt 240
   cccgccccgt cctgcgcgag ctgcctgccc aggcaggttc gccggtgcga gcgtaaaggg 300
   gcatccctat cagtgataga ccatccctat cagtgataga ccatccctat cagtgataga 360
   ccggagctag gactgccttg ggcggtataa atagcaggga accgcgcggt cgctcagcag 420
   tgacgtgaca cgcagcccac ggtctgtact gacgcgccct cgcttcttcc tctttctcga 480
   ctccatcttc gcggtagctg ggaccgccgt tcaggtaaga atggggcctt ggctgcagcc 540
   gaagggcttg tagcaggttg gctgcggggt cagaaggcgc ggggggaacc gaagaacggg 600
   gcctgctccg tggccctgct ccagtcccta tccgaactcc ttgggaggcc tggccttccc 660
   cacgtgagcc gccgcgacca ccatcccgtc gcgatcgttt ctggaccgct ttccactccc 720
   aaatctcctt tatcccagag catttcttgg cttctcttac aagccgtctt ttctttactc 780
   agtcgccgtc gac 793
<210> 9
   <211> 238
   <212> DNA
   <213> Homo sapiens
<400> 9
   gatgttgtga ggacatgtgg aagcacttta agttttttca tcataacata aattattttc 60
   aagtgtaact tattaaccta tttattattt atgtatttat ttaagcatca aatatttgtg 120
   caagaatttg gaaaaataga agatgaatca ttgattgaat agttataaag atgttatagt 180
   aaatttattt tattttagat attaaatgat gttttattag ataaatttca atcagggt 238
<210> 10
   <211> 250
   <212> DNA
   <213> Homo sapiens
<400> 10
   aagacctcac ctagaaattg acacaagtgg accttaggcc ttcctctctc cagatgtttc 60
   cagacttcct tgagacacgg agcccagccc tccccatgga gccagctccc tctatttatg 120
   tttgcacttg tgattattta ttatttattt attatttatt tatttacaga tgaatgtatt 180
   tatttgggag accggggtat cctgggggac ccaatgtagg agctgccttg gctcagacat 240
   gttttccgtg 250